(19) Europäisches Patentamt
European Patent Office
Office européen des brevets

(11) **EP 1 685 248 B1**

(12) **EUROPÄISCHE PATENTSCHRIFT**

(45) Veröffentlichungstag und Bekanntmachung des Hinweises auf die Patenterteilung:
**09.06.2010 Patentblatt 2010/23**

(21) Anmeldenummer: **04818808.0**

(22) Anmeldetag: **19.11.2004**

(51) Int Cl.:
*C12N 15/53* (2006.01)     *C12N 9/04* (2006.01)
*C12P 41/00* (2006.01)     *C12N 15/62* (2006.01)
*C12N 5/10* (2006.01)     *C07K 16/40* (2006.01)

(86) Internationale Anmeldenummer:
**PCT/EP2004/013156**

(87) Internationale Veröffentlichungsnummer:
**WO 2005/049816 (02.06.2005 Gazette 2005/22)**

(54) **OXIDOREDUKTASE AUS METSCHNIKOWIA ZOBELLII**

OXIDOREDUCTASE FROM METSCHNIKOWIA ZOBELLII

OXYDOREDUCTASE DE METSCHNIKOWIA ZOBELLII

(84) Benannte Vertragsstaaten:
**AT BE BG CH CY CZ DE DK EE ES FI FR GB GR HU IE IS IT LI LU MC NL PL PT RO SE SI SK TR**

(30) Priorität: **21.11.2003 DE 10354779**

(43) Veröffentlichungstag der Anmeldung:
**02.08.2006 Patentblatt 2006/31**

(73) Patentinhaber: **IEP GmbH**
**65203 Wiesbaden (DE)**

(72) Erfinder:
• **GUPTA, Antje**
**65207 Wiesbaden (DE)**
• **BOBKOVA, Maria**
**65510 Idstein (DE)**
• **ZIMMER, Anke**
**65205 Wiesbaden (DE)**

(74) Vertreter: **Schwarz, Albin**
**Schwarz & Partner**
**Patentanwälte**
**Wipplingerstraße 30**
**1010 Wien (AT)**

(56) Entgegenhaltungen:
**EP-A- 0 918 090     US-A1- 2003 064 432**

• **COSTELLO C A ET AL: "Purification, characterization, cDNA cloning and expression of a novel ketoreductase from Zygosaccharomyces rouxii." EUROPEAN JOURNAL OF BIOCHEMISTRY / FEBS. SEP 2000, Bd. 267, Nr. 17, September 2000 (2000-09), Seiten 5493-5501, XP002328959 ISSN: 0014-2956**
• **DATABASE UniProt [Online] 25. Oktober 2004 (2004-10-25), "Similar to tr Q12068 Saccharomyces cerevisiae YOL151w GRE2." XP002328960 gefunden im EBI accession no. UNIPROT:Q6BYX1 Database accession no. Q6BYX1**
• **WADA M ET AL: "PURIFICATION AND CHARACTERIZATION OF NADPH-DEPENDENT CARBONYL REDUCTASE, INVOLVED IN STEREOSELECTIVE REDUCTION OF ETHYL 4-CHLORO-3-OXOBUTANOATE, FROM CANDIDA MAGNOLIAE" BIOSCIENCE BIOTECHNOLOGY BIOCHEMISTRY, JAPAN SOC. FOR BIOSCIENCE, BIOTECHNOLOGY AND AGROCHEM, TOKYO, JP, Bd. 62, Nr. 2, Februar 1998 (1998-02), Seiten 280-285, XP001094913 ISSN: 0916-8451**

## Beschreibung

Die vorliegende Erfindung betrifft eine neue NADPH abhängige Oxidoreduktase aus Hefen, beispielsweise der Gattung Metschnikowia, insbesondere aus Metschnikowia zobellii, ein enzymatisches Verfahren zur enantioselektiven Reduktion organischer Ketoverbindungen zu den entsprechenden Hydroxyverbindungen und ein enzymatisches Verfahren zur enantioselektiven Gewinnung von Hydroxyverbindungen im Zwei-Phasen-System unter Verwendung der isolierten rekombinant überexprimierten Oxidoreduktase aus Metschnikowia zobellii.

Optisch aktive Hydroxyverbindungen sind wertvolle chirale Bausteine mit breiter Anwendung für die Synthese von pharmakologisch wirksamen Verbindungen, aromatischer Substanzen, Pheromonen, Agrochemikalien oder Enzyminhibitoren.

Die Zahl der für großtechnische Anwendungen in der Biokatalyse geeigneter und in ausreichendem Maße preiswert zur Verfügung stehender Carbonylreduktasen ist dabei sehr begrenzt. Bei den bisher in Form von isolierten Enzymen einsetzbaren Alkoholdehydrogenasen handelt es sich weitgehend um sekundäre Alkoholdehydrogenasen, deren Substratspektrum auf Substrate mit einer großen und einer kleinen Seitenkette in Nachbarschaft zur Carbonylfunktion beschränkt ist, wobei die maximale Länge der kleineren der beiden Seitenketten drei C-Atome betragen darf.

Solche Enzyme sind beispielsweise

Alkoholdehydrogenase aus Pferdeleber (HLADH) (Enzyme Engineering, Vol 6, 1982, Seite 107).

Alkoholdehydrogenase aus Hefe (YADH) (Alcohol dehydrogenases: The Enzymes, (1963) Seiten 25-83. New York: Academic Press),
Carbonylreduktase aus Candida parapsilosis (CPCR) (US 5,523,223 und US 5,763,236) oder Pichia capsulata ADH (DE10327454.4).
Carbonylreduktase aus Rhodococcus erythropolis (RECR) (US 5,523,223) und Norcardia fusca (Biosci. Biotechnol. Biochem.,63 (10) (1999), Seiten 1721-1729),
Alkohodehydrogenase aus Candida boidinii (Biochim,. Biophys. Acta 716, (1982), Seiten 298-307) oder
Alkoholdehydrogenase aus Sulfolobus solfataricus (FEMS Microbiology Letters, 170 (1999), Seiten 31-39).

R-spezifische sekundären Alkoholdehydrogenasen aus Organismen der Gattung Lactobacillus (Lactobacillus kefir (US5200335), Lactobacillus brevis (DE 19610984 A1), Lactobacillus minor (DE10119274) oder Pseudomonas (US 05385833).

Des weiteren werden noch Enzyme aus den Organismen Candida magnoliae (WO 98/35025), Kluyveromyces lactis (JP-A Hei 11-187869) und Sporobolomyces salmonicolor (FEMS Microbiology Letters 70 (1990) 45-48) zur Ketoredukion verwendet. Bei diesen Enzymen handelte es sich um Homologe zur Untereinheit des Fettsäuresynthasekomplexes deren Anwendung bisher auf die Reduktion von 4-Chloracetoacetat beschränkt blieb.

Wenige der genannten Carbonylreduktasen haben bisher großtechnisch Anwendung gefunden. Der Hauptgrund hierfür ist neben den oft zu engen Substratspektren oder der geringen Enantioselektivität der Enzyme vor allem die Verfügbarkeit dieser Enzyme. Die meisten der genannten Enzyme konnten bisher nicht rekombinant in ausreichendem Maße zu günstigen Preisen zur Verfügung gestellt werden.

Es wurde nun gefunden, dass durch eine neue Oxidoreduktase die genannten Nachteile der Verfahren aus dem Stand der Technik behoben werden können. Bei der erfindungsgemäßen Oxidoreduktase handelt es sich um eine neuartige Oxidoreduktase die keinerlei Verwandtschaft oder Homologie zu bereits bekannten Carbonylreduktasen aufweist.

Die EP 0 918 090 A beschreibt eine Carbonylreduktase, die aus Zygosaccharomyces rouxii gewonnen wird und Ketonsubstrate in Anwesenheit von NADPH und Wasser enantioselektiv zu den entsprechenden Hydroxyverbindungen reduziert.

Von Costello C.A. et al. (Eur. J. Biochem. 267, 2000: 5493-5501) wird ebenfalls eine aus Zygosaccharomyces rouxii isolierte Ketoreduktase beschrieben, welche die asymmetrische Reduktion bestimmter Ketonsubstrate mit NADPH als Coenzym katalysiert.

Die Stereoselektivität der erfindungsgemäßen Oxidoreduktase wird dabei nicht nur durch die Größe der die Carbonylgruppe flankierenden Seitenketten sondern auch durch deren Hydrophobizität bestimmt. Dadurch unterscheiden sich Substratspektrum und Enantioselektivität der erfindungsgemäßen Oxidoreduktase deutlich von der bekannter Enzymklassen.

Die erfindungsgemäße Oxidoreduktase ist **dadurch gekennzeichnet, dass** sie in Anwesenheit von NADPH und Wasser Carbonylverbindung zu den entsprechenden chiralen Hydroxyverbindungen reduziert und mehr als 70 % der Aminosäuren identisch sind mit der Aminosäuresequenz SEQ ID NO: 13 und sie eine spezifische Aktivität von mehr als 1 μmol pro mg Protein aufweist, bezogen auf die Umsetzung von 2-Oxo-4-phenylbuttersäureethylester zu (R)-2-Hydroxy-4-phenylbuttersäureethylester. Sie kann beispielsweise aus Hefen der Gattung Metschnikowia, insbesondere aus Metschnikowia zobellii gewonnen werden.

Bevorzugt ist eine Oxidoreduktase aus Metschnikowia zobellii, die die DNA-Sequenz gemäß SEQ ID NO: 12 und die Aminosäuresequenz gemäß SEQ ID NO: 13 aufweist, wie im anliegenden Sequenzprotokoll beschrieben.

Bevorzugt ist eine Oxidoreduktase, in der 80 % bis 99,5 %, insbesondere 90 % bis 99,5 %, insbesondere bevorzugt 99 % bis 99,5 % der Aminosäuren identisch sind mit der Aminosäuresequenz von SEQ ID NO: 13. Die Messung der spezifischen Aktivität der Oxidoreduktase gemäß SEQ ID NO: 13 oder seiner wie nachfolgend definierten Derivate oder Analogons erfolgt mit dem Testsystem, das in Beispiel 1 beschrieben wird.

Geeignet sind auch Oxidoreduktasen, die 1 bis 40 Aminosäuren zusätzlich oder 1 bis 40 Aminosäuren weniger aufweisen als die Oxidoreduktase mit der Aminosäuresequenz SEQ ID NO: 13 und die eine spezifische Aktivität von mehr als 1 $\mu$mol pro mg Protein zeigt, bezogen auf die Umsetzung von 2-Oxo-4-phenylbuttersäureethylester zu (R)-2-Hydroxy-4-phenylbuttersäureethytester. Bevorzugt sind die Oxidoreduktasen, in denen 1 bis 25 Aminosäuren, insbesondere 2 bis 20 Aminosäuren, insbesondere bevorzugt 3 bis 10 Aminosäuren mehr oder weniger in der Aminosäuresequenz SEQ ID NO: 13 vorkommen.

Verwendbar ist auch eine Oxidoreduktase, die die Aminosäuresequenz von SEQ ID NO: 13 aufweist und ein-, zwei-, drei-, vier- oder fünffach derivatisiert ist durch ein wasserlösliches Polymer und die eine spezifische Aktivität von mehr als 1 $\mu$mol pro mg Protein aufweist, bezogen auf die Umsetzung von 2-Oxo-4-phenylbuttersäureethylester zu (R)-2-Hydroxy-4-phenylbuttersäureethylester. Ein wasserlösliches Polymer ist beispielsweise Polyethylenglykol. Die Bindung des Polyethylenglykols erfolgt bevorzugt am N-terminalen Ende des Proteins gemäß SEQ ID NO: 13. Die Oxidoreduktase gemäß SEQ ID NO: 13 kann auch an einen Festkörper, bevorzugt aus Polyethylen, Polystyrol, Polysaccharid, Cellulose oder einem Cellulosederivat, gebunden sein.

Die Erfindung betrifft ferner Proteinfragmente der Aminosäuresequenz SEQ ID NO: 13 mit 5 bis 30 Aminosäuren je Fragment. Bevorzugt sind Fragmente von SEQ ID NO: 13 mit einer Kettenlänge von 6 bis 25 Aminosäuren, bevorzugt 8 bis 20 Aminosäuren, besonders bevorzugt 10 bis 18 Aminosäuren. Diese Fragmente können beispielsweise zum Auffinden der erfindungsgemäßen Oxidoreduktase aus Metschnikowia zobellii oder aus beliebig anderen Mikroorganismen eingesetzt werden.

Die Erfindung betrifft ferner ein Fusionsprotein, das die Oxidoreduktase mit der Aminosäuresequenz SEQ ID NO: 13 oder ein Fragment davon mit 5 bis 30 Aminosäuren umfaßt, die mit einem weiteren Polypeptid am N-terminalen oder Carboxy-terminalen Ende peptidisch verbunden sind. Fusionsproteine können beispielsweise leichter von anderen Proteinen abgetrennt werden oder werden in einer größeren Menge in den Zellen exprimiert.

Die Erfindung betrifft ferner Antikörper, die spezifisch an die Oxidoreduktase gemäß SEQ ID NO: 13 binden. Die Herstellung dieser Antikörper erfolgt nach bekannten Methoden durch Immunisierung von geeigneten Säugetieren und anschließender Gewinnung der Antikörper. Die Antikörper können monoklonal oder polyklonal sein.

Die Erfindung betrifft auch die (isolierte) Nukleinsäuresequenz, die für die Oxidoreduktase gemäß SEQ ID NO: 13 kodiert.

Die Erfindung betrifft ferner die (isolierte) Desoxyribonukleinsäuresequenz (DNA-Sequenz) der Oxidoreduktase, die die Reduktion von Carbonylverbindung in Anwesenheit von NADPH und Wasser zu den entsprechenden Hydroxyverbindungen katalysiert, wobei die DNA-Sequenz ausgewählt wird aus der Gruppe

a) DNA-Sequenz, welche die Nukleotidsequenz gemäß SEQ ID NO: 12, SEQ ID NO: 7 oder SEQ ID NO: 8 aufweist oder der jeweils komplementäre Strang,

b) DNA-Sequenz, welche mit einer oder mehrerer der DNA-Sequenzen gemäß a) oder seiner komplementären Stränge hybridisiert, wobei die Hybridisierung unter stringenten Bedingungen wie beschrieben (Sambrok and Russel, Molecular Cloning a laboratory Manual , Vol 1, Chapter 1 Protocol 30-32) erfolgt, und

c) DNA-Sequenz, welche auf Grund der Degeneration des genetischen Codes, ein Protein kodiert, das durch eine oder mehrere der DNA-Sequenzen gemäß a) oder b) kodiert wird.

Die Erfindung betrifft ferner eine DNA-Sequenz, in der mehr als 70 % der Nukleinsäurebasen identisch sind mit der DNA-Sequenz gemäß SEQ ID NO: 12 oder deren komplementären Stränge und die für eine Oxidoreduktase kodiert, die eine spezifische Aktivität von mehr als 1 $\mu$mol pro mg Protein aufweist, bezogen auf die Umsetzung von 2-Oxo-4-phenyl-buttersäureethylester zu (R)-2-Hydroxy-4-phenyl-buttersäureethylester. Bevorzugt sind DNA-Sequenzen, in denen 80 % bis 99,5 %, bevorzugt 90 % bis 99,5 %, besonders bevorzugt 99 % bis 99,5 %, der Nukleinsäurebasen identisch sind mit der DNA-Sequenz gemäß SEQ ID NO: 12.

Die Erfindung betrifft ferner einen Klonierungsvektor, enthaltend eine oder mehrere der obengenannten Nukleinsäure- oder DNA-Sequenzen. Die Erfindung betrifft ferner einen Expressionsvektor, der sich in einer Bakterien-, Insekten-, Pflanzen- oder Tierzelle (insbesondere Säugetierzelle) befindet und eine oder mehrere der obengenannten Nukleinsäure- oder DNA-Sequenzen enthält, die in geeigneter Weise mit einer Expressionskontrollsequenz verbunden sind. Die Erfindung betrifft ferner eine Wirtszelle, die eine Bakterien-, Hefe-, Insekten-, oder Pflanzen zelle ist und mit einem der obengenannten Expressionsvektoren transformiert oder transfektiert wurde.

Die Identitäten der vorgenannten DNA-Sequenzen oder Aminosäuresequenz werden dadurch berechnet, dass man die Anzahl der Aminosäuren oder Nukleinsäurebasen summiert, die mit Teilsequenzen der jeweiligen Proteine oder DNA-Sequenzen identisch sind, durch die Gesamtzahl der Aminosäuren oder Nukleinsäurebasen dividiert und mit Hundert multipliziert.

Geeignete Klonierungsvektoren sind beispielsweise ppCR-Script, pCMV-Script , pBluescript (Stratagene), pDrive cloning Vector (Qiagen, Hilden, Deutschland), pS Blue, pET Blue, pET LIC-Vektoren (Novagen, Madison, USA) sowie TA-PCR Klonierungsvektoren (Invitrogen, Karlsruhe, Deutschland).

Geeignete Expressionsvektoren sind beispielsweise pKK223-3, pTrc99a, pUC, pTZ, pSK, pBluescript, pGEM, pQE, pET, PHUB, pPLc, pKC30, pRM1/pRM9. pTrxFus,pAS1, pGEx, pMAL oder pTrx.

Geeignete Expressionskontrollsequenzen sind beispielsweise trp-lac (tac)-Promotor, trp-lac (trc) promotor, lac-Promotor, T7-Promotor oder λpL-Promotor.

Die Oxidoreduktase aus Metschnikowia zobellii ist ein Monomer mit einem Molekulargewicht bestimmt im SDS-Gel von 36 $\pm$ 2 kDa. Das Temperatur-optimum der Oxidoreduktase liegt im Bereich von 20 °C bis 25 °C und das pH-Optimum für die Reduktionsreaktion liegt im Bereich von 5,5 bis 6,5. Die Oxidoreduktase aus Metschnikowia zobellii ist im pH-Bereich von 5,5 bis 8,5 und im Temperaturbereich von 15 °C bis zu 30 °C für 5 Stunden stabil und zeigt ferner eine gute Stabilität in organischen Lösungsmitteln.

Das Enzym ist insbesondere aus Hefen der Gattung Metschnikowia isolierbar und kann im spektrophotometrischen Test über die Abnahme von NADPH bei 340 nm in Gegenwart eines entsprechenden Substrates, beispielsweise 2-Oxo-4-phenyl-buttersäureethylester, nachgewiesen werden.

Eine erfindungsgemäße Oxidoreduktase aus Metschnikowia zobellii wurde kloniert und konnte in Escherichia coli (E. coli) mit Aktivitäten von 1000 bis 10 000 U/g E. coli Feuchtgewicht überexprimiert werden. Das Enzym ist somit preiswert und in großen Mengen verfügbar.

Sequenzvergleiche in Datenbanken ergaben, dass die Oxidoreduktase aus Metschnikowia zobellii keine direkte Homologie zu bekannten Enzymen aufweist, eine entfernte Verwandschaft konnte zur Gruppe der Hydroxysteroiddehydrogenasen festgestellt werden.

Die Erfindung betrifft auch ein Verfahren zur Gewinnung der Oxidoreduktase aus Metschnikowia zobellii. Dazu wird die DNA, die für die Oxidoreduktase aus Metschnikowia zobellii kodiert, beispielsweise in einem geeigneten prokaryotischen oder eukaryotischen Mikroorganismus exprimiert. Bevorzugt wird die Oxidoreduktase aus Metschnikowia zobellii in einen E. coli Stamm transformiert und exprimiert, insbesondere in E. coli BL21star (DE3) Zellen (Invitrogen, Katalognummer C6010-03).

Die Oxidoreduktase aus Metschnikowia zobellii lässt sich beispielsweise so gewinnen, dass die oben genannten rekombinanten E. coli Zellen kultiviert werden, die Expression der Oxidoreduktase induziert wird und anschließend nach etwa 10 bis 18 Stunden die Zellen durch Ultraschallbehandlung oder durch Naßvermahlung mit Glasperlen in einer Kugelmühle (Retsch, GmbH, Haan Deutschland 10 min, 24 Hz) aufgeschlossen werden. Der erhaltene Zellextrakt kann entweder direkt verwendet oder weiter gereinigt werden. Dazu wird der Zellextrakt beispielsweise zentrifugiert und der erhaltene Überstand wird einer hydrophoben Interaktionschromatographie unterworfen, beispielsweise an Octylseparose Fast Flow® (Pharmacia).

Die Erfindung betrifft ferner ein Verfahren zur enantioselektiven Reduktion von Carbonylverbindungen zu den entsprechenden chiralen Hydroxyverbindungen, das dadurch gekennzeichnet ist, dass man

a) eine Carbonylverbindung in Anwesenheit der erfindungsgemäßen Oxidoreduktase, NADPH und Wasser zur entsprechenden chiralen Hydroxy-verbindung reduziert, und

b) die gebildete chirale Hydroxyverbindung isoliert.

Das erfindungsgemäße Verfahren weist eine hohe Standzeit auf, eine enantiomeren Reinheit von mehr als 90 % der hergestellten chiralen Hydroxyverbindungen und eine hohe Ausbeute bezogen auf die eingesetzte Menge der Carbonylverbindung.

Unter dem Begriff "NADPH" wird reduziertes Nicotinamid-adenin-dinucleotidphosphat verstanden. Unter dem Begriff "NADP" wird Nicotinamid-adenindinucleotid-phosphat verstanden.

Unter dem Begriff "Carbonylverbindung" werden beispielsweise Verbindungen der Formel I

$$R1-C(O)-R2 \qquad (I)$$

verstanden.

R1 steht beispielsweise für

1) $(C_1-C_{20})$Alkyl, das geradkettig oder verzweigt ist,
2) $(C_2-C_{20})$Alkenyl, das geradkettig oder verzweigt ist und, je nach Kettenlänge, ein, zwei, drei oder vier Dop-

pelbindungen enthält,

3) (C$_2$-C$_{20}$)Alkinyl, das geradkettig oder verzweigt ist und, je nach Kettenlänge, ein, zwei, drei oder vier Dreifachbindungen enthält,

4) (C$_6$-C$_{14}$)Aryl,

5) (C$_1$-C$_8$)Alkyl-(C$_6$-C$_{14}$)Aryl,

6) einen (C$_5$-C$_{14}$)Heterocyclus, der unsubstituiert oder ein- bis dreifach substituiert ist, bevorzugt durch Halogen, Hydroxyl, Amino oder Nitro, oder

7) (C$_3$-C$_7$)Cycloalkyl,

wobei die oben unter 1) bis 7) genannten Reste unsubstituiert sind oder ein-, zwei- oder dreifach substituiert sind unabhängig voneinander durch

a) -OH,

b) Halogen, wie Fluor, Chlor, Brom oder Jod,

c) -NO$_2$ oder

d) -NH$_2$.

R2 steht beispielsweise für

1) (C$_1$-C$_6$)Alkyl, das geradkettig oder verzweigt ist,

2) (C$_2$-C$_6$)Alkenyl, das geradkettig oder verzweigt ist und, je nach Kettenlänge, ein, zwei oder drei Doppelbindungen enthält,

3) (C$_2$-C$_6$)Alkinyl, das geradkettig oder verzweigt ist und, je nach Kettenlänge, ein oder zwei Dreifachbindungen enthält, oder

4) (C$_0$-C$_{10}$)Alkyl-C(O)-O-(C$_1$-C$_6$)Alkyl, worin Alkyl gerade oder verzweigt ist und unsubstituiert oder ein- bis dreifach substituiert ist durch Halogen, Hydroxyl, Amino oder Nitro,

wobei die oben unter 1) bis 4) genannten Reste unsubstituiert sind oder ein-, zwei- oder dreifach substituiert sind unabhängig voneinander durch

a) -OH,

b) Halogen, wie Fluor, Chlor, Brom oder Jod,

c) -NO$_2$ oder

d) -NH$_2$.

Unter dem Begriff chirale Hydroxyverbindung werden beispielsweise Verbindungen der Formel II

$$R1\text{-}C(OH)\text{-}R2 \qquad (II)$$

verstanden, wobei die -OH Gruppe in Abhängigkeit der Reste R1 und R2 in R-oder in S-Konfiguration zum Kohlenstoffatom steht an das sie gebunden ist und R1 und R2 dieselbe Bedeutung wie in Formel I haben.

Unter dem Begriff Aryl werden aromatische Kohlenstoffreste verstanden mit 6 bis 14 Kohlenstoffatomen im Ring. (C$_6$-C$_{14}$)Arylreste sind beispielsweise Phenyl, Naphthyl, zum Beispiel 1-Naphthyl, 2-Naphthyl, Biphenylyl, zum Beispiel Biphenyl-2-yl, Biphenyl-3-yl und Biphenyl-4-yl, Anthracenyl oder Fluorenyl. Biphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "(C$_1$-C$_{20}$)Alkyl wird ein Kohlenwasserstoffrest verstanden, dessen Kohlenstoffkette geradkettig oder verzweigt ist und 1 bis 20 Kohlenstoffatome enthält. Beispiele sind Methyl, Ethyl, Propyl, Isopropyl, Butyl, tert.-Butyl, Pentyl, Hexyl, Heptyl, Octyl, Nonyl oder Decyl. Unter "C$_0$-Alkyl" soll im Zusammenhang mit der vorliegenden Erfindung eine kovalente Bindung verstanden werden.

Unter dem Begriff "(C$_3$-C$_7$)Cycloalkyl" werden cyclische Kohlenwasserstoffreste verstanden wie Cyclopropyl, Cylobutyl, Cyclopentyl, Cyclohexyl oder Cycloheptyl.

Der Begriff "(C$_5$-C$_{14}$)Heterocyclus" steht für einen monocyclischen oder bicyclischen, 5-gliedrigen bis 14-gliedrigen heterocyclischen Ring, der teilweise oder vollständig gesättigt ist. Beispiele für Heteroatome sind N, O und S. Beispiele für die Begriffe (C$_5$-C$_{14}$)Heterocyclus sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, die durch F, -CN, -CF$_3$ oder -CO-O-(C$_1$-C$_4$)Alkyl substituiert sein können, 3-Hydroxy-pyrrolidin-2,4-dione, 5-Oxo-1,2,4-thiadiazole, Pyridin, Pyrazin, Pyrimidin, Indol, Isoindol, Indazol, Phthalazin, Chinolin, Isochinolin, Chinoxalin, Chinazolin, Cinnolin, Carbolin und benzanellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]pyrrol-5-yl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder Dihydropyridinyl, Pyrrolidinyl, zum Beispiel 2- oder 3-(N-Methyl-pyrrolidinyl), Piperazinyl, Morpholinyl, Thiomorpholinyl, Tetrahydrothienyl oder Benzodioxolanyl.

Bevorzugte Verbindungen der Formel I sind beispielsweise 2-Oxo-4-phenylbuttersäureethylester, Ethylphenylglyoxylat, 1-Phenyl-2-propanon, 2,3-Dichloracetophenon, 2-Octanon, 8-Chlor-6-oxo-octansäureethylester, 3-Oxo-octan-1,8-disäuredimethylester, 2-Oxo-cyclohexancarbonsäureethylester, 2-Methyl-cyclohexanon oder 3-Methylcyclohexanon .

Die entsprechend gebildeten S-Alkohole sind beispielsweise (R)-2-Hydroxy-4-phenylbuttersäureethylester, Ethyl-(R)-mandelat, (S)-2-Octanol oder (R)-Ethyl-8-chlor-6-hydroxy-octansäure, (S)-1-Phenyl-2-propanol oder (R)-2-Chlor-1-(3-chlorphenyl)-ethan-1-ol.

Geeignete Oxidoreduktasen stammen beispielsweise aus Metschnikowia zobellii. Die Oxidoreduktase kann in dem erfindungsgemäßen Verfahren entweder vollständig gereinigt oder teilweise gereinigt eingesetzt werden oder mit Zellen, enthaltend die erfindungsgemäße Oxidoreduktase, durchgeführt werden. Die eingesetzten Zellen können dabei nativ, permeabilisiert oder lysiert vorliegen. Bevorzugt wird die klonierte Oxidoreduktase gemäß SEQ ID NO: 13 eingesetzt.

Die Volumenaktivität der eingesetzten Oxidoreduktase beträgt von 100 Units/ml (U/ml) bis 5000 U/ml, bevorzugt etwa 500 U/ml.

Je kg umzusetzender Verbindung der Formel I werden 5000 bis 2 000 000 U Oxidoreduktase eingesetzt, bevorzugt etwa 10 000 bis 500 000 U. Der Enzymeinheit 1 U entspricht dabei der Enzymmenge die benötigt wird um 1 $\mu$mol der Verbindung der Formel I je Minute (min) umzusetzen.

Die Erfindung betrifft ferner ein Verfahren zur enantioselektiven Reduktion von Carbonylverbindungen zu den entsprechenden chiralen Hydroxyverbindungen, das dadurch gekennzeichnet ist, dass man

> a) eine Carbonylverbindung in Anwesenheit von Oxidoreduktase, NADPH und Wasser zum entsprechenden chiralen Hydroxyverbindung reduziert,
> b) das durch die Oxidoreduktase gebildete NADP mit einer Dehydrogenase und einem Cosubstrat gleichzeitig zu NADPH reduziert, und
> c) die gebildete chirale Hydroxyverbindung isoliert.

Geeignete Dehydrogenasen sind beispielsweise sekundäre Alkohol-Dehydrogenasen aus Thermoanaerobium brockii, Lactobacillus kefir oder Lactobacillus brevis, wobei diese Enzyme als Coenzym NADPH benötigen (DE 19 610 984, EP 0 456 107, WO 97/32012).

Geeignete Cosubstrate für die eingesetzte Alkohol-Dehydrogenase sind Alkohole wie 2-Propanol (Isopropanol), 2-Butanol, 2-Pentanol oder 2-Octanol.

Ferner kann die NADP-Reduktion auch mit den bekannten zur Regenerierung von NADPH verwendeten Enzymen durchgeführt werden, beispielsweise mit Glucose-Dehydrogenase oder NADPH abhängiger Formiat-Dehydrogenase (Tishkov et al., J. Biotechnol. Bioeng. [1999] 64, 187-193, Pilot-scale production and isolation of recombinant NAD and NADP specific formate dehydrogenase).

Das geeignte Cosubstrat für das erfindungsgemäße Verfahren ist bei Einsatz der Glucose-Dehydrogenase Glucose. Geeignete Cosubstrate der Formiat-Dehydrogenase sind beispielsweise Salze der Ameisensäure wie Ammoniumformiat, Natriumformiat oder Calciumformiat.

Bevorzugt werden die Alkoholdehydrogenasen aus Lactobacillus minor (DE 101 19274) oder Thermoanerobium brockii eingesetzt.

Die Alkohol-Dehydrogenase kann in dem erfindungsgemäßen Verfahren entweder vollständig gereinigt oder teilweise gereinigt eingesetzt werden oder es können ganze Zellen, die die Alkohol-Dehydrogenase enthalten, verwendet werden. Die eingesetzten Zellen können dabei nativ, permeabilisiert oder lysiert vorliegen.

Je kg umzusetzender Verbindung der Formel I werden 10 000 U bis 2 000 000 U Alkohol-Dehydrogenase eingesetzt, bevorzugt etwa 20 000 U bis 500 000 U. Der Enzymeinheit 1 U entspricht dabei der Enzymmenge die benötigt wird um 1 $\mu$mol des Cosubstrates (z.B. 2-Propanol) je Minute (min) umzusetzen.

Dem Wasser wird bevorzugt ein Puffer zugesetzt, beispielsweise Kaliumphosphat-, Tris/HCl- oder Triethanolamin-Puffer mit einem pH-Wert von 5 bis 10, vorzugsweise ein pH-Wert von 6 bis 9. Die Pufferkonzentration beträgt von 10 mM bis 200 mM, bevorzugt von 50 mM bis 150 mM, insbesondere 100 mM.

Der Puffer kann zusätzlich noch Ionen zur Stabilisierung oder Aktivierung beider Enzyme enthalten, beispielsweise Magnesiumionen zur Stabilisierung der Alkohol-Dehydrogenase aus Lactobaillus minor.

Weiterhin kann der Puffer Detergenzien enthalten, beispielsweise ®Triton X100, Triton X45, Triton X114, Triton X450 zur Reaktionsvermittlung.

Die Temperatur beträgt bei den erfindungsgemäßen Verfahren beispielsweise von etwa 10 °C bis 30 °C, bevorzugt von 20°C bis 25 °C.

Die Erfindung betrifft ferner ein Verfahren zur enantioselektiven Reduktion von Carbonylverbindungen zu den entsprechenden chiralen Hydroxyverbindungen, das dadurch gekennzeichnet ist, dass man

a) eine Carbonylverbindung in Anwesenheit der erfindungsgemäßen Oxidoreduktase, NADPH und Wasser zur entsprechenden chiralen Hydroxy-verbindung reduziert,

b) das durch die Oxidoreduktase gebildete NADP mit einer Dehydrogenase und einem Cosubstrat gleichzeitig zu NADPH reduziert,

c) die Reaktionen in Anwesenheit eines organischen Lösungsmittel durchführt und

d) die gebildete chirale Hydroxyverbindung isoliert.

Die bevorzugten organischen Lösungsmittel sind beispielsweise tertiär-Butylmethylether, Diisopropylether, Heptan, Hexan oder Cyclohexan oder deren Gemische unterschiedlicher Zusammensetzung.

Der Reaktionsansatz besteht beim Einsatz zusätzlicher Lösungsmittel allgemein aus einer wäßrigen Phase und einer organischen Phase. Die organische Phase wird durch ein geeignetes Lösungsmittel in dem das Substrat gelöst vorliegt oder wird durch das wasserunlösliche Substrat selbst gebildet. Die organische Phase hat dabei allgemein einen Anteil von etwa 5 bis 80 %, bevorzugt 10 bis 40 %, bezogen auf das gesamte Reaktionsvolumen.

Das Wasser bildet im Zwei-Phasen-System die eine flüssige Phase und das organische Lösungsmittel bildet die zweite flüssige Phase. Gegebenenfalls kann auch noch eine feste oder weitere flüssige Phase vorliegen, die beispielsweise durch nicht vollständig gelöste Oxidoreduktase und/oder Alkohol-Dehydrogenase oder durch die Verbindung der Formel I entsteht. Bevorzugt sind jedoch zwei flüssige Phasen ohne feste Phase. Die zwei flüssigen Phasen werden bevorzugt mechanisch gemischt, so dass große Oberflächen zwischen den beiden flüssigen Phasen erzeugt werden.

Die Konzentration des Cofaktors NADPH bezogen auf die wässrige Phase beträgt von 0,001 mM bis 1 mM, insbesondere von 0,01 mM bis 0,1 mM.

Bevorzugt wird im erfindungsgemäßen Verfahren noch ein Stabilisator der Alkohol-Dehydrogenase eingesetzt. Geeignete Stabilisatoren sind beispielsweise Glycerin, Sorbit oder Dimethylsulfoxid (DMSO).

Die Verbindungen der Formel I werden im erfindungsgemäßen Verfahren in einer Menge von 2 % bis 30 % (w/v) bezogen auf das Gesamtvolumen eingesetzt, bevorzugt von 5% bis 20 % (w/v), insbesondere 8 % bis 15 % (w/v).

Die Menge des Cosubstrats für die Regenerierung von NADP zu NADPH wie Isopropanol beträgt von etwa 2 % bis 30 % bezogen auf das Gesamtvolumen, bevorzugt von 5 % bis 20 % , insbesondere von 8 % bis 15 %.

Das erfindungsgemäße Verfahren wird beispielsweise in einem geschlossen Reaktionsgefäß aus Glas oder Metall durchgeführt. Dazu werden die Komponenten einzeln in das Reaktionsgefäß überführt und Rühren unter einer Atmosphäre von beispielsweise Stickstoff oder Luft gerührt. Je nach Substrat und eingesetzter Verbindung der Formel I beträgt die Reaktionszeit von 1 Stunde bis 96 Stunden, insbesondere von 2 Stunden bis 24 Stunden.

Anschließend wird das Reaktionsgemisch aufgearbeitet. Dazu wird die wässrige Phase abgetrennt, die organische Phase wird gefiltert. Die wässrige Phase kann gegebenenfalls noch einmal extrahiert werden und wie die organische Phase weiter aufgearbeitet werden. Danach wird gegebenenfalls das Lösungsmittel aus der klaren organischen Phase verdampft. Man erhält so beispielsweise das Produkt (R)-2-Hydroxy-4-phenylbuttersäureethylester mit einer Enantiomerenreinheit von mehr als 90 %. Die Gesamtausbeute der Prozesses beträgt nach Destillation des Produktes etwa 50 % bis 95 %, bezogen auf die Menge Ausgangsmaterials.

Die Erfindung soll mit folgenden Beispielen erläutert werden:

Beispiel 1

Screening von Hefen nach Carbonylreduktasen zur Darstellung von (R)-2-Hydroxy-4-phenylbuttersäureethylester

Zum Screening wurden eine Reihe verschiedene Hefestämme in folgendem Medium kultiviert: Die Angaben in der Klammer beziehen sich jeweils auf g pro Liter Wasser: Hefeextrakt (3), Malzextrakt (3), Peptone (5) und Glucose (10).

Das Medium wurde bei 121 °C sterilisiert und die Hefen wurden ohne weitere pH-Regulierung bei 25 °C und auf einem Schüttler bei 160 Umdrehungen pro Minute (rpm) kultiviert.

Anschließend wurden 125 mg Zellen mit 800 μl Aufschlusspuffer (100 mM Triethanolamin (TEA), pH = 7,0) resuspendiert, mit 1 g Glasperlen versetzt und 10 Minuten (min) bei 4 °C und 24 Hz in der Kugelmühle aufgeschlossen (Retsch). Der nach 2 min Zentrifugation bei 12000 rpm erhaltene Überstand (Lysat) wurde im folgenden Aktivitätsscreening und zur Bestimmung des Enantiomerenüberschusses (ee-Wert) eingesetzt. Als Substrat wurde 2-Oxo-4-phenyl-buttersäureethylester eingesetzt.

Ansatz für das Aktivitätsscreening:

| 860 µl | 0,1 M TEA pH = 7,0 |
|---|---|
| 20 µl | NADPH oder NADH (10 mM) |
| 20 µl | Lysat |
| 100 µl | 2-Oxo-4-phenyl-buttersäureethylester (10 mM) |

Die Reaktion wurde 1 min bei 340 nm verfolgt.

Ansatz für die ee-Wert-Bestimmung:

| 20 µl | Lysat |
|---|---|
| 100 µl | NADPH (50 mM) |
| 2 µl | 2-Oxo-4-phenyl-buttersäureethylester |

Die Ansätze zur ee-Bestimmung wurden nach 24 Stunden (h) mit Chloroform extrahiert und mittels Gaschromatographie (GC) wurde der Enantiomerenüberschuss bestimmt.

Der Enantiomerenüberschuss wurde wie folgt berechnet:

$$ee(\%) = ((R\text{-Alkohol} - S\text{-Alkohol})/(R\text{-Alkohol} + S\text{-Alkohol})) \times 100.$$

Tabelle 1

| DSMZ Nr. | Mikrorganismen-Name | 2-Oxo-4-phenylbuttersäureethylester Aktivität in U/g Zellen Wirtsorganismus | | | |
|---|---|---|---|---|---|
| | | NADH | NADPH | ee-Wert NADH | ee-Wert NADPH |
| 1345 | Yarrowia lipolytika | 0 | 3,4 | ---- | 32 % S |
| 3434 | Kluyveromyces thermotolerans | 0 | 7,1 | ---- | 60 % S |
| 3435 | Metschnikowia zobellii | 0 | 11,6 | ---- | 86 % R |
| 3795 | Kluyveromyces lactis | 0 | 5,4 | ---- | rac |
| 70130 | Pichia anomala | 0 | 3 | ---- | 20 % S |
| 70277 | Pichia angusta | 0 | 6,4 | ---- | 30 % R |
| 70382 | Pichia pastoris | 0 | 3,2 | ---- | 70 % R |
| 70638 | Candida magnoliae | 0 | 14,5 | ---- | 24 % R |
| 70125 | Candida parapsilosis | 0 | 6,4 | ---- | 60 % R |
| 2147 | Pichia methanolica | 0 | 7,1 | --- | 73 % S |
| 70260 | Pichia capsulata | 19 | 4,8 | ---- | 40 % S |

DSMZ steht für Deutschen Sammlung für Mikroorganismen und Zellkulturen, Mascheroder Weg 1 b, 38124 Braunschweig

Definition der Enzymeinheiten: 1 U entspricht der Enzymmenge die benötigt wird um 1 µmol Substrat pro min umzusetzen.

Beispiel 2

Isolierung einer NADPH-abhängigen Oxidoreduktase aus Metschnikowia zobellii

Zur Isolierung der NADPH abhängigen Oxidoreduktase aus Metschnikowia zobellii wurde der Mikroorganismus wie unter Beispiel 1 beschrieben kultiviert. Nach Erreichen der stationären Phase wurden die Zellen geerntet und durch Zentrifugation vom Medium getrennt. Die Enzymfreisetzung erfolgte durch Nassvermahlung mittels Glasperlen, kann aber auch durch andere Aufschlussmethoden erreicht werden. Dazu wurden 66 g Metschnikowia zobellii mit 264 ml Aufschlusspuffer (100 mM Triethanolamin, pH =7,0) suspendiert und mittels einer French press homogenisiert.

Der nach Zentrifugation (7000 rpm) erhaltene Rohextrakt wurde dann mittels FPLC (fast protein liquid chromatography) weiter aufgereinigt.

Die erfindungsgemäße Oxidoreduktase konnte durch Kombination von hydrophober Interaktionschromatographie an Octylsepharose Fast Flow, Butylsepharose HiTrap (Pharmacia) und Ionenaustauschchromatographie an Uno Q (Biorad, München, Deutschland) in drei Schritten aufgereinigt werden.

Dazu wurde das nach Zentrifugation erhaltene Lysat direkt auf eine mit 100 mM Triethanolaminpuffer pH = 7,0 1 M $(NH_4)_2SO_4$ äquilibrierte Octylsepharose FF-Säule aufgetragen und mit fallendem linearen Salzgradienten eluiert. Die Oxidoreduktase wurde dabei bei 0,2 bis 0,3 M $(NH_4)_2SO_4$ eluiert. Die oxidoreduktase-enthaltenden Fraktionen wurden vereinigt und mittels Ultrafiltration (Ausschlussgrenze 10 kDa) auf ein geeignetes Volumen eingeengt.

Anschließend wurde die eingeengten Fraktionen der Oxidoreduktase mittels Uno Q weiter aufgereinigt. Dazu wurde die Oxidoreduktase direkt auf eine mit 50 mM Kaliumphosphatpuffer pH = 7,0 äquilibrierte UnoQ-Säule (Biorad) aufgetragen und mit steigendem linearen Salzgradienten eluiert, wobei die Oxidoreduktase ohne Bindung bei 0 M NaCl eluierte, während ein Großteil der Verunreinigungen gebunden wurde und bei höheren Salzkonzentrationen eluiert werden konnte.

Der dritte Reinigungsschritt wurde an einer Butylsepharose HiTrap Säule (Pharmacia) durchgeführt, wobei dieselben Puffer wie beim ersten Reinigungsschritt eingesetzt wurden und die Oxidoreduktase bei einer Konzentration von 0,6 bis 0,5M $(NH_4)_2SO_4$ eluierte.

Danach wurde das Molekulargewicht der erhaltenen aufgereinigten Oxidoreduktase mit Hilfe von Gelpermeation (Superdex 200 HR; Pharmacia, 100 mM Triethanolamin, pH = 7, 0,15 M NaCl ) bestimmt. Als Molekulargewichtsstandards wurden Catalase (232 kDa), Aldolase (158 kDa), Albumin (69,8kDa) und Ovalbumin (49,4 kDa) verwendet.

Die folgende Tabelle 2 fasst die erhaltenen Ergebnisse zusammen:

Tabelle 2:

| Reinigungsschritt | Volumen [ml] | Aktivität [U/ml] | Gesamt-aktivität [U] | Spezifische Aktivität [U/mg] | Ausbeute |
|---|---|---|---|---|---|
| Rohextrakt | 270 | 1,85 | 500 | 0,12 | 100 % |
| Octylsepharose | 18 | 5 | 90 | 4,3 | 18 % |
| Uno Q | 1,8 | 41 | 75 | 59 | 15 % |
| Butylsepharose HiTrap | 1,5 | 13 | 19,5 | 130 | 4 % |

Die Enzymaktivität der Oxidoreduktase wurde im Testsystem gemäß Beispiel 1, (Ansatz Aktivitätsscreening, Messung mit NADPH) bestimmt und die Bestimmung der Proteinmenge erfolgte gemäß Lowry et al. Journal of Biological Chemistry 193 (1951) 265-275 oder Peterson et al., Analytical Biochemistry, 100 (1979) 201-220). Der Quotient von Enzymaktivität zu Proteinmenge ergibt die spezifische Aktivität, wobei der Umsatz von 1 μmol pro min 1 Unit (U) entspricht.

Das mittels Gelpermation bestimmte Molekulargewicht der erfindungsgemäßen Oxidoreduktase betrug im nativen Zustand 36 ± 2 kDa.

Beispiel 3

Bestimmung zweier interner Peptide nach In-Gelverdau

Die SDS-PAGE- Bande des gereinigten Proteins wurde mit Dithiothreitol reduziert, carboxymethyliert und mit Endoproteinase Lys-C verdaut. Die erhaltenen Peptide wurden über eine 300 μm x 150 mm Kapillar-HPLC-Säule (Vydac RP18, LC Packings getrennt. Fraktionen wurden manuell gesammelt und mittels MALDI MS (Voyager-DE STR (PE-Biosystems) auf für die Sequenzierung geeignete Peptide geprüft. Zwei geeignete Fraktionen wurden mittels automatischem Edman-Abbau sequenziert und lieferte folgende Sequenzen:

SEQ ID 1:
ELLLPAVEGTK

SEQ ID 2:
LGPNDEVPSQ(SHW)(GSC)(G)FVDV

Beispiel 4

Klonierung der Oxidoreduktase aus Metschnikowia zobellii

Der eukaryotische Mikrorganismus Metschnikowia zobellii gehört zu der Familie Metschnikowiaceae. Die Genomstruktur dieses Organismus weist eine Exon-Intron Anordnung auf. Daher wurde für die Identifizierung der Gensequenz, die für die enantioselektive Oxidoreduktase kodiert, eine cDNA Bibliothek aus den aktiven Zellen der Metschnikowia zobellii angelegt.

4.1 Präparation der gesamten RNA aus den Zellen von Metschnikowia zobellii

600 mg frische Zellen von Metschnikowia zobellii wurden in 2,5 ml eiskalten LETS Puffer (10 mM Tris-HCl, pH = 7,4, 10 mM EDTA, 100 mM LiCl, 0,2 % SDS) resuspendiert. Zu der Zellsuspension wurden 5 ml (etwa 20 g) in Salpetersäure gewaschener und mit 3 ml Phenol (pH 7.0) equilibrierte Glasperlen zugegeben. Der gesamte Ansatz wurde dann abwechselnd jeweils 30 Sekunden (sec) geschüttelt (Vortex Genie 2, Scientific Industries Inc., New York, USA) und 30 sec auf Eis gekühlt und insgesamt 10 min behandelt. Anschließend wurden weitere 5 ml eiskalter LETS Puffer dazugegeben. Diese Zellsuspension wurde für 5 min bei 11000 g bei 4 °C zentrifugiert. Die wässrige Phase wurde abgenommen und mit dem gleichen Volumen an Phenol: Chloroform: 3-Methyl-1-butanol (24:24:1) zweimal extrahiert. Anschließend erfolgte eine Extraktion mit Chloroform. Nach der letzten Extraktion wurde die gesamte erhaltene RNA durch die Zugabe von 1/10 Vol von 5 M LiCl bei -20 °C für 4 Stunden präzipitiert.
1 mg der oben isolierten RNA wurde für die m-RNA Anreicherung mittels OligodT Cellulose (mRNA PräpKit, Qiagen) eingesetzt.

4.2 Herstellung einer cDNA Bibliothek ausgehend von der mRNA aus Metschnikowia zobellii

Nach der anschließenden Präzipitation wurden 5 μg mRNA für die cDNA Synthese (pBluescript IIXR cDNA Library Construction kit, Stratagene) verwendet. Die nach den Angaben des Herstellers konstruierte Bibliothek wurde in XL-10 Gold E. coli transformiert und mit einem Titer von 4 x $10^6$ colony forming units (cfu) nach der blau-weiß Selektion etabliert.

4.3 Identifizierung der für eine NADPH-abhängige Oxidoreduktase kodierenden cDNA

4.3.1 Identifizierung des N-Terminus.

Ausgehend von den Peptidsequenzen Seq ID 1 und Seq ID 2 wurden an Metschnikowia Codon Nutzung angepasste degenerierte Primer abgeleitet (Seq. 3; Seq. 4; Seq. 5; Seq. 6) und in die Polymerase-Ketten Reaktion eingesetzt. Dabei diente aus XL-10 Gold Zellen isolierte Plasmid cDNA Bibliothek als Matrize für die erste PCR Runde. Da cDNA Bibliothek als eine Plasmid Bibliothek etabliert wurde, war es möglich auch stationären Primer in Kombination mit abgeleiteten, degenerierten Primern in die PCR Reaktion einzusetzen. Dabei wurden Primer T3 und T7 verwendet, die jeweils an die 5'- bzw. 3'- cDNA-Inserts flankierende Sequenz im Plasmid Rückgrad binden.

Seq ID 3 = Oligo MBme1-for: 5 '- CCNGCHGTDGAAGGWACWAAR - 3'

Seq ID 4 = Oligo MBme1-rev1: 5'- YTTWGTWCCTTCHACDGCMGG - 3'

Seq ID 5 = Oligo MBme2-for: 5'- GGNCCNAAYGATGARGTNCC - 3'

Seq ID 6 = Oligo MBme2-rev: 5' - GGNACNTCATCRTTNGGRCC - 3'

Seq ID 14 = Oligo T3-for: 5' - AATTAACCCTCACTAAAGGG - 3'

Seq ID 15 = Oligo T7-rev: 5'- TAATACGACTCACTATAGGG - 3'

Die PCR-Reaktion wurde in folgendem Reaktionsgemisch durchgeführt: 10 mM Tris-HCl (pH 8,3), 50 mM KCl, 2 mM MgCl$_2$, 1 mM dNTP Mix, 30 pMol je Primer MBme2-rev und T3-for und 2.5 U AmpliTaq Gold (Applied Biosystems)

Nach einer Aktivierung der AmpliTaq Gold Polymerase (5 min, 95 °C) und folgenden 25 Zyklen PCR (95° C, 45 sec; 55 °C, 45 sec; 70 ° C, 45 sec) wurde die Reaktion auf 4° C abgekühlt und teils auf ein 1 % Agarose Gel zur Analyse aufgetragen.

In einer darauffolgenden PCR Reaktion wurde das Amplifizierungssignal durch "nested" PCR verstärkt. Dabei wurden in die Polymerase-Ketten-Reaktion die Primer MBme1-rev und T3-for eingesetzt. Als Matrize dienten 2 μl PCR Reaktion aus der ersten Reaktion. Die Reaktion wurde in einem PCR Puffer [10 mM Tris-HCl (pH 8.3), 50 mM KCl, 2 mM MgCl$_2$, 1mM dNTP Mix, 30 pMol je Primer MBme1-rev und T3-for und 2,5 U AmpliTaq Gold (Applied Biosystems) durchgeführt.

Ein spezifisches Fragment von 500 bp wurde identifiziert, mittels Qiagen Gel Extraktionskit aufgereinigt und in pCR2.1 Vektor (Invitrogen) zwecks nachfolgender Sequenzierung ligiert. Die Analyse des somit entstandenen Plasmids lieferte eine cDNA Sequenz, die dem N-Terminus der gesuchten Oxidoreduktase entsprach (Seq. ID 7) und die N-terminale Aminosäuresequenz Seq ID 16 aufwies.

4.3.1 Identifizierung des C-Terminus.

Für die Identifizierung des C-Terminus des gesuchten Oxidoreduktase-Gens wurde eine Polymerase-Ketten-Reaktion mit den Primern MBme2-for und T7 durchgeführt. Als Matrize diente die Plasmid Präparation aus der XL-10 Gold cDNA Plasmid Bibliothek. Die Amplifizierungsreaktion wurde in folgendem Reaktionsgemisch bei 4 verschiedenen Bindungstemperaturen (53 °C; 55 °C; 57 °C; 59 °C) durchgeführt.

Reaktionsgemisch: 10 mM Tris-HCl (pH 8,3), 50 mM KCl, 2 mM MgCl$_2$, 1 mM dNTP Mix, 30 pMol je Primer MBme2-for und T7-rev und 2.5 U AmpliTaq Gold (Applied Biosystems)

Nach einer Aktivierung der AmpliTaq Gold Polymerase (5 min, 95 °C) und folgenden 25 Zyklen PCR (95° C, 45 sec; 55 °C, 45 sec; 70 ° C, 45 sec) wurde die Reaktion auf 4 °C abgekühlt und teils auf ein 1 % Agarose Gel zur Analyse aufgetragen.

Eine spezifische Bande einer Größe von 500 Basenpaaren (bp) wurde in allen Ansätzen der Gradienten PCR identifiziert. Nach einer Aufreinigung aus dem Gel mittels Qiagen Gel Extraktionskit wurde das identifizierte PCR Fragment für eine nachfolgende Sequenzierung in den Vektor pCR2.1 (Invitragen) ligiert. Die Analyse der Nukleotidfolge dieses Fragmentes lieferte die Sequenz, die dem C-Terminus der gesuchten Oxidoreduktase entsprach (Seq. ID 8) und die C-terminale Aminosäuresequenz Seq ID 17 aufwies.

4.4 Herstellung des Expressionskonstruktes für die rekombinante Produktion der Oxidoreduktase von Metschnikowia zobellii

Basierend auf Sequenzen No: 7 und No: 8 wurden spezifische Primer für eine Klonierung des Volllänge-Gens in ein passendes Expressionssystem konstruiert. Dabei wurden 5'-Primer mit einer Erkennungssequenz für *Nde I* und 3'-Primer mit einer Erkennungssequenz für *Hind III* für die Klonierung in den pET21 a Expressionsvektor modifiziert (Seq. 9, Seq. 10). Für die Klonierung in Vektoren der pQE Serie wurde der 5' Primer mit einer Erkennungssequenz für die Bam HI Restriktions-endonuclease versehen (Seq. 11).

Die im Plasmid pBluescript (Stratagene) etablierte cDNA Bibliothek von Metschnikowia zobellii diente als Matrize für die Polymerase Ketten-Reaktion. Die PCR-Reaktion wurde in einem PCR-Puffer [10 mM Tris-HCl, (pH 8,0); 50 mM KCl; 10 mM MgSO$_4$; 1 mM dNTP Mix; je 30 pMol Primer und 2.5 U Platinum Pfx DNA-Polymerase (Invitrogen)] mit 300 ng Template und folgenden TemperaturZyklen durchgeführt:

| | |
|---|---|
| Zyklus 1: | 94 ° C, 2 min |
| Zyklus 2 x 30: | 94 ° C, 15 sec |
| | 58 ° C, 30 sec |
| | 68 ° C, 75 sec |
| Zyklus 3: | 68 ° C, 7 min |
| | 4 ° C, ∞ |

Das Amplifikat (etwa 1000 bp) wurde nach der Reinigung über ein 1 % Agarose Gel mit Hilfe der Endonukleasen *Nde I* und *Hind III*, oder mit den Endonucleasen *Bam HI* und *Hind III* verdaut, und in das mit gleichen Endonukleasen behandelten Rückrad des pET21a Vectors (Novagen), oder pQE30 Vectors (Qiagen) ligiert. Nach der Transformation von 2 μl des Ligationsansatzes in E. coli Top 10 F' Zellen (Invitrogen) wurden Plasmid-DNA's Ampicillin-resistenter Kolonien mittels einer Restriktionsanalyse mit Endonukleasen *Nde I* und *Hind III*, oder Endonucleasen *Bam HI* und *Hind*

*III* auf das Vorhandensein eines 1000 bp großen Inserts getestet. Der Expressionskonstrukt pET21-MEvl#1 und pQE-30 -Mevl #9 wurden sequenziert. Das für eine Oxidoreduktase kodierende Transkript aus Metschnikowia zobellii besitzt einen offenen Leserrahmen von insgesamt 1014 bp (Seq. 12) was einem Protein von 338 Aminosäuren entspricht. (Seq. 13).

4.5 Produktion der rekombinanten Oxidoreduktase aus Metschnikowia zobellii in Escherichia coli

4.5.1 BL 21 (De3) Zellen

Kompetente Escherichia coli StarBL21(De3) Zellen (Invitrogen) wurden mit dem für die erfindungsgemäße Oxidoreduktase kodierendem Expressionskonstrukt pET21-MEvl#1 transformiert.
Der Stamm wurde in LB Medium kultiviert (1% Trypton, 0,5 % Hefeextrakt, 1 % NaCl, Ampicillin 50 $\mu$g/ml) und bei Erreichen einer optischen Dichte von 0,5, gemessen bei 500 nm, mit 1 mM IPTG (Isopropylthiogalaktosid) induziert. Die erreichte Aktivität betrug 4000 U / g Biofeuchtgewicht.

4.5.2 E.coli RB791

Kompetente Escherichia coli RB791 Zellen wurden mit dem für die erfindungsgemäße Oxidoreduktase kodierendem Expressionskonstrukt pQE30-MEvl#9 transformiert.
Der Stamm wurde in LB Medium kultiviert (1% Trypton, 0,5 % Hefeextrakt, 1 % NaCl, Ampicillin 50 $\mu$g/ml) und bei Erreichen einer optischen Dichte von 0,5, gemessen bei 500 nm, mit 1 mM IPTG (Isopropylthiogalaktosid) induziert.
Die erreichte Aktivität betrug 320 U / g Biofeuchtgewicht.

Beispiel 5

Charakterisierung der rekombinanten Oxidoreduktase aus Metschnikowia zobellii

5.1 pH-Optimum

Es wurden die i n Tabelle 3 aufgeführten Puffer hergestellt. Die Konzentration der jeweiligen Pufferkomponenten betrug jeweils 50 mM.

Tabelle 3

| PH-Wert | Puffersystem | pH-Wert | Puffersystem |
|---|---|---|---|
| 4 | Na-acetat/Essigsäure | 7,5 | $KH_2PO_4/K_2HPO_4$ |
| 4,5 | Na-acetat/Essigsäure | 8 | $KH_2PO_4/K_2HPO_4$ |
| 5 | Na-acetat/Essigsäure | 8,5 | $KH_2PO_4/K_2HPO_4$ |
| 5,5 | $KH_2PO_4/K_2HPO_4$ | 9 | Glycin/NaOH |
| 6 | $KH_2PO_4/K_2HPO_4$ | 9,5 | Glycin/NaOH |
| 6,5 | $KH_2PO_4/K_2PO_4$ | 10 | Glycin/NaOH |
| 7 | $KH_2PO_4/K_2HPO_4$ | 11 | Glycin/NaOH |

Messansatz (25 °C):

|  |  |  |
|---|---|---|
| 870 | $\mu$l | der jeweils in Tabelle 3 genannten Puffersysteme |
| 20 | $\mu$l | NADPH 10 mM (8,6 mg/ml Wasser) |
| 10 | $\mu$l | Enzym verdünnt |

Es wurde etwa 2 bis 3 min inkubiert, danach erfolgte die Zugabe von

|  |  |  |
|---|---|---|
| 100 | $\mu$l | Substratlösung (100mM 2-Oxo-4-phenyl-buttersäureethylester). |

Zur Bestimmung des pH-Optimums wurde die enzymatische Reaktion in dem jeweiligen in Tabelle 3 aufgeführten Puffer bestimmt. Zur Bestimmung des pH-Optimums für die Reduktion wurde als Cofaktor NADPH und als Substrat 2-Oxo-4-phenylbuttersäureethylester eingesetzt. Dabei konnte für das erfindungsgemäße Enzym ein pH-Optimum von 5,5 bis 6,5 für die Reduktionsreaktion ermittelt werden.

5.2.1 Temperatur-Optimum

Zur Bestimmung der optimalen Testtemperatur wurde die Enzymaktivität im Temperaturbereich von 15 °C bis 70 °C im Standardmessansatz gemessen. Wie aus Tabelle 4 ersichtlich hat das Enzym seine maximale Aktivität bei 25 °C, anschließend sinkt die Aktivität rapide ab.

Tabelle 4

| Temperatur (°C) | Aktivität in U/ml unverdünntes Enzym | Temperatur (°C) | Aktivität in U/ml unverdünntes Enzym |
|---|---|---|---|
| 15 | 115 | 45 | 0 |
| 20 | 86 | 50 | 0 |
| 25 | 135 | 55 | 0 |
| 30 | 112 | 60 | 0 |
| 35 | 0 | 65 | 0 |
| 40 | 0 | 70 | 0 |

5.3 Temperatur-Stabilität

In analoger Weise wie unter Beispiel 5.2 beschrieben wurde die Temperatur-stabilität für den Bereich von 15 °C bis 70 °C bestimmt. Dazu wurde jeweils eine 1:20 Verdünnung der gereinigten Oxidoreduktase für 60 min und 180 min bei der jeweiligen Temperatur inkubiert und anschließend bei 30 °C mit dem obigen Testansatz gemessen. Auch hier wurde als Ausgangswert der Messwert herangezogen, den man unmittelbar nach Verdünnung der gereinigten Oxidoreduktase in Kali umphosphatpuffer 50 mM bei pH = 7,0 erhält. Dieser Wert wurde auch in diesem Beispiel als 100%-Wert gesetzt.
Das Enzym erwies sich dabei bei Temperaturen unter 25 °C als stabil über mehrere Tage während die Enzymaktivität bei Temperaturen über 25 °C innerhalb von wenigen Stunden rapide abnimmt.

5.4 Substratspektrum/Enantiomerenüberschuss

Das Substratspektrum der erfindungsgemäßen Oxidoreduktase wurde durch Messung der Enzymaktivität mit einer Reihe von Ketonen, Oxosäureestem und cyclischen Ketonen bestimmt. Dazu wurde der Standard Messansatz gemäß Beispiel 5.1 mit unterschiedlichen Substraten verwendet Die Aktivität mit 2-Oxo-4-phenyl-buttersäureethylester wurde gleich 100% gesetzt und alle anderen Substrate wurden dazu ins Verhältnis gesetzt.
Für die ee-Wert-Bestimmung wurden für ausgewählte Substrate folgender Reaktionsansatz verwendet:

| 100 | µl | TEA 100 mM pH = 7 |
|---|---|---|
| 100 | µl | NADPH (50 mM) |
| 60 | µl | Substrat (100 mM) |

+ 1 bis 2 U der erfindungsgemäßen Oxidoreduktase

Die Ansätze zur ee- Bestimmung wurden nach 24 h mit Chloroform extrahiert und mittels GC wurde der Enantiomerenüberschuss des resultierenden Alkohols bestimmt.

Tabelle 5

| Substrat | Relative Aktivität % | Stereo-selektivität | Substrat | Relative Aktivität % | Stereo-selektivität |
|---|---|---|---|---|---|
| 1-Phenyl-2-propanon | 15 % | 97 % S | 4-Chloracetessigsäureethylester | 100 % | rac |
| 2-chlor-1-(3-chlor-pheny)-ethan-1-on | 5 % | 97 % R | 8-Chlor-6-oxo-octansäure-ethylester | 50 % | 100 % R |
| Acetophenon | 0 % | n. b. | 3-Oxo-1,8-octandisäure-dimethylester | 35 % | n.b. |
| Caprylophenon | 0 % | n.b. | Cyclohexanon | 3 % | n.b. |
| Butan-2-on | 0 % | n.b. | 2-Oxo-cyclohexan-carbonsäure-ethylester | 20 % | n. b. |
| 1,4-Dichlor-butan-2-on | 27 % | 40 % S | 2-Methylcyclohexanon | 8 % | n.b. |
| Ethyl-2-oxovaleriansäure | 100 % | 62 % R | 3-Methylcyclohexanon | 8 % | n.b. |
| Ethyl-2-oxo-4-phenyl-buttersäure | 100 % | 95 % R | (2-chlorphenyl)-oxo-essigsäure ethylester | 50 % | 99,5 % R |
| Ethyl-brompyruvat | 28 % | n.b. | 2-Oxo-cyclopentan carbonsäure-ethylester | 8 % | n.b. |
| Ethylphenylglyoxylat | 50 % | 99,5 % R | | | |
| n.b. bedeutet nicht bestimmt | | | | | |

Wie aus Tabelle 5 ersichtlich, werden von der erfindungsgemäßen Oxidoreduktase ein breites Spektrum an aliphatischen und zyklischen Carbonylverbindungen, insbesondere 2-Oxosäureestern enantioselektiv reduziert. Kurzkettige sekundäre Alkohole werden nicht akzeptiert. Das bedeutet, dass hier die substratgekoppelte Coenzymregenerierung mit sekundären Alkoholen als Cosubstrat nicht angewendet werden kann.

5.5 Umsetzungsbeispiele

5.5.1 Reduktion von 2-Oxo-4-phenylbuttersäureethylester zu (R)-2-Hydroxy-4-phenyl-buttersäureethylester

Zur Reduktion von 2-Oxo-4-phenylbuttersäureethylester wurde ein Gemisch aus 800 μl Puffer (100 mM TEA, pH = 8, 10 % ®Triton X 100), 100 μl Isopropanol (1,3, mmol), 0,1 mg NADP (0,1 μmol) 10 U rekombinante Oxidoreduktase aus Metschnikowia zobellii und 5 Units Alkoholdehydrogenase aus Thermoanerobium brockii mit 50 μl 2-Oxo-4-phenyl-buttersäureethylester (0,25 mmol) gelöst in 600 μl n-Heptan überschichtet und für 24 h bei 25 °C unter ständiger Durchmischung inkubiert.
Nach 24 h waren 92% des eingesetzten 2-Oxo-4-phenyl-buttersäureethylester reduziert. Der Enantiomerenüberschuss des erhaltenen (R)-2-Hydroxy-4-phenyl-buttersäureethylester betrug mehr als 90 %.

5.5.2. Reduktion von 8-Chlor-6-oxo-octansäureethylester zu (R)-8-Chlor-6-hydroxy-octansäureethylester

Zur Reduktion von 8-Chloro-6-oxo-octansäureethylester wurde ein Gemisch aus 800 μl Puffer (100mM TEA, pH = 8, 5 % Triton X 100), 80 μl Isopropanol (1,04 mmol), 0,1 mg NADP (0,1 μmol) 25 U rekombinante Oxidoreduktase aus Metschnikowia zobellii und 5 Units Alkoholdehydrogenase aus Thermoanerobium brockii mit 50 μl 8-Chlor-6-oxo-octansäureethylester (0,23 mmol) gelöst in 600 μl Cyclohexan überschichtet und für 24 h bei 25 °C unter ständiger Durchmischung inkubiert.
Nach 24 h waren 55% des eingesetzten 8-Chlor-6-oxo-octansäureethylesters reduziert. Der Enantiomerenüberschuss des erhaltenen (R)-8-Chlor-6-hydroxy-octansäureethylesters betrug mehr als 97 % .

5.5.3. Reduktion von 2-Chlor-1-phenyl-ethan-1-on zu (1R)-2-Chlor-1-phenyl-ethan-1-ol

Zur Reduktion von 2-Chlor-1-phenyl-ethan-1-on wurde ein Gemisch aus 800 µl Puffer (100mM TEA, pH = 8, 5 % Triton X 100), 100 µl Isopropanol (1,3 mmol), 0,1 mg NADP (0,1 µmol) 20 U rekombinante Oxidoreduktase aus Metschnikowia zobellii und 10 Units Alkoholdehydrogenase aus Thermoanerobium brockii mit 50 mg 2-Chlor-1-phenyl-ethan-1-on (0,33 mmol) gelöst in 400 µl Cyclohexan/tert.-Butyl-methylether (50:50) überschichtet und für 24 h bei 25 °C unter ständiger Durchmischung inkubiert.

Nach 48 h waren 75% des eingesetzten 2-Chlor-1-phenyl-ethan-1-ons reduziert. Der Enantiomerenüberschuss des erhaltenen (1R)-2-Chlor-1-phenyl-ethan-1-ols betrug mehr als 94 %.

5.5.4. Reduktion von Ethylphenylglyoxylat zu (1 R)-Ethylmandelat

Zur Reduktion von Ethylphenylglyoxylat wurde ein Gemisch aus 800 µl Puffer (100mM TEA, pH = 8, 5 % Triton X 100), 100 µl Isopropanol (1,3 mmol), 0,1 mg NADP (0,1 µmol) 20 U rekombinante Oxidoreduktase aus Metschnikowia zobellii und 10 Units Alkoholdehydrogenase aus Thermoanerobium brockii mit 50 µl Ethylphenylglyoxylat (0,31 mmol) gelöst in 600 µl Cyclohexan überschichtet und für 24 h bei 25 °C unter ständiger Durchmischung inkubiert.

Nach 48 h waren 50% des eingesetzten Ethylphenylglyoxylat reduziert. Der Enantiomerenüberschuss des erhaltenen (1R)-Ethylmandelat betrug mehr als 99%.

**Patentansprüche**

1.  Oxidoreduktase, welche in Anwesenheit von NADPH und Wasser eine Carbonylverbindung zur entsprechenden chiralen Hydroxyverbindung reduziert, **dadurch gekennzeichnet, dass** mehr als 70 % ihrer Aminosäuren mit der Aminosäuresequenz SEQ ID NO: 13 identisch sind und dass sie eine spezifische Aktivität von mehr als 1 μmol pro mg Protein aufweist, bezogen auf die Umsetzung von 2-Oxo-4-phenyl-buttersäureethylester zu (R)-2-Hydroxy-4-phenyl-buttersäureethylester.

2.  Oxidoreduktase gemäß Anspruch 1, **dadurch gekennzeichnet, dass** 80 bis 99,5 %, bevorzugt 90 bis 99,5 %, besonders bevorzugt 99 bis 99,5 %, der Aminosäuren mit der Aminosäuresequenz SEQ ID NO: 13 identisch sind.

3.  Oxidoreduktase gemäß Anspruch 1 oder 2, **dadurch gekennzeichnet, dass** sie die Aminosäuresequenz SEQ ID NO: 13 aufweist und durch die DNA-Sequenz SEQ ID NO: 12 kodiert wird.

4.  Oxidoreduktase gemäß einem der Ansprüche 1 bis 3, **dadurch gekennzeichnet, dass** sie aus Hefen der Gattung Metschnikowia, insbesondere aus Metschnikowia zobellii, erhältlich ist.

5.  Oxidoreduktase gemäß einem der Ansprüche 1 bis 4, **dadurch gekennzeichnet, dass** sie 1 bis 40 Aminosäuren zusätzlich zur oder 1 bis 40 Aminosäuren weniger aufweist als die Aminosäuresequenz SEQ ID NO: 13 und daß sie eine spezifische Aktivität von mehr als 1 μmol pro mg Protein aufweist, bezogen auf die Umsetzung von 2-Oxo-4-phenylbuttersäureethylester zu (R)-2-Hydroxy-4-phenyl-buttersäureethylester.

6. Oxidoreduktase gemäß Anspruch 5, **dadurch gekennzeichnet, dass** sie 1 bis 25 Aminosäuren, insbesondere 2 bis 20 Aminosäuren, besonders bevorzugt 3 bis 10 Aminosäuren, mehr oder weniger als die Aminosäuresequenz SEQ ID NO: 13 aufweist.

7. Oxidoreduktase gemäß einem der Ansprüche 1 bis 6, **dadurch gekennzeichnet, dass** sie die Aminosäuresequenz SEQ ID NO: 13 aufweist, ein-, zwei-, drei-, vier- oder fünffach durch ein wasserlösliches Polymer modifiziert ist und eine spezifische Aktivität von mehr als 1 μmol pro mg Protein zeigt, bezogen auf die Umsetzung von 2-Oxo-4-phenyl-buttersäureethylester zu (R)-2-Hydroxy-4-phenyl-buttersäureethylester.

8. Oxidoreduktase gemäß Anspruch 7, **dadurch gekennzeichnet, dass** das wasserlösliche Polymer Polyethylenglykol ist.

9. Antikörper, **dadurch gekennzeichnet, dass** er spezifisch an die Oxidoreduktase gemäß SEQ ID NO: 13 bindet.

10. DNA-Sequenz, welche für die Oxidoreduktase gemäß SEQ ID NO: 13 kodiert.

11. DNA-Sequenz, welche für eine Oxidoreduktase kodiert, die in Anwesenheit von NADPH und Wasser die Reduktion einer Carbonylverbindung zur entsprechenden chiralen Hydroxyverbindung katalysiert, wobei die DNA-Sequenz

   a) SEQ ID NO: 12 oder deren komplementärem Strang entspricht oder
   b) eine DNA-Sequenz ist, die mit der DNA-Sequenz gemäß a) oder deren komplementärem Strang hybridisiert, wobei die Hybridisierung unter stringenten Bedingungen erfolgt, oder
   c) eine DNA-Sequenz ist, die aufgrund der Degeneration des genetischen Codes ein Protein kodiert, wie es auch durch eine DNA-Sequenz gemäß a) oder b) kodiert wird.

12. DNA-Sequenz, **dadurch gekennzeichnet, dass** darin mehr als 70 % der Nukleinsäurebasen mit der DNA-Sequenz SEQ ID NO: 12 oder deren komplementärem Strang identisch sind und daß sie eine Oxidoreduktase kodiert, die eine spezifische Aktivität von mehr als 1 μmol pro mg Protein aufweist, bezogen auf die Umsetzung von 2-Oxo-4-phenyl-buttersäureethylester zu (R)-2-Hydroxy-4-phenyl-buttersäureethylester.

13. DNA-Sequenz gemäß Anspruch 12, **dadurch gekennzeichnet, dass** 80 % bis 99,5 %, bevorzugt 90 % bis 99,5 %, besonders bevorzugt 99 % bis 99,5 %, der Nukleinsäurebasen mit der DNA-Sequenz SEQ ID NO: 12 identisch sind.

14. Klonierungsvektor, **dadurch gekennzeichnet, dass** er eine oder mehrere der DNA-Sequenzen gemäß einem der Ansprüche 10 bis 13 umfaßt.

15. Expressionsvektor, **dadurch gekennzeichnet, dass** er eine oder mehrere der DNA-Sequenzen gemäß einem der Ansprüche 10 bis 13 umfaßt und mit einer Expressionskontrollsequenz verbunden ist.

16. Wirtszelle, die eine Bakterien-, Hefe-, Insekten-, oder Pflanzen zelle, ist und mit einem Expressionsvektor gemäß Anspruch 15 transformiert oder transfektiert wurde.

17. Verfahren zur enantioselektiven Reduktion von Carbonylverbindungen zu den entsprechenden chiralen Hydroxyverbindungen, **dadurch gekennzeichnet, dass**

   a) eine Carbonylverbindung in Anwesenheit einer Oxidoreduktase gemäß einem der Ansprüche 1 bis 8, NADPH und Wasser zur entsprechenden chiralen Hydroxyverbindung reduziert, und
   b) die gebildete chirale Hydroxyverbindung isoliert wird.

18. Verfahren gemäß Anspruch 17, **dadurch gekennzeichnet, daß** als Carbonylverbindung eine Verbindung der Formel I einsetzt wird,

$$R1\text{-}C(O)\text{-}R2 \qquad (I)$$

worin R1 für

1) $(C_1-C_{20})$Alkyl, das geradkettig nd er verzweigt ist,
2) $(C_2-C_{20})$Alkenyl, das geradkettig oder verzweigt ist und je nach Kettenlänge ein, zwei, drei oder vier Doppelbindungen enthält,
3) $(C_2-C_{20})$Alkinyl, das geradkettig oder verzweigt ist und je nach Kcttenlänge ein, zwei, drei oder vier Dreifachbindungen enthält,
4) $(C_6-C_{14})$Aryl,
5) $(C_1-C_8)$Alkyl-$(C_6-C_{14})$Aryl,
6) $(C_5-C_{14})$Heterocyclus, der unsubstituiert oder ein- bis dreifach substituiert ist, bevorzugt durch Halogen, Hydroxyl, Amino oder Nitro, oder
7) $(C_3-C_7)$Cycloalkyl,
wobei die oben unter 1) bis 7) genannten Reste unsubstituiert sind oder ein-, zwei- oder dreifach substituiert sind unabhängig voneinander durch

a) -OH,
b) Halogen, wie Fluor, Chlor, Brom oder Jod,
c) $-NO_2$ oder
d) $-NH_2$ steht, und

R2 für

1) $(C_1-C_6)$Alkyl, das geradkettig oder verzweigt ist,
2) $(C_2-C_6)$Alkenyl, das geradkettig oder verzweigt ist und, je nach Kettenlänge, ein, zwei oder drei Doppelbindungen enthält,
3) $(C_2-C_6)$Alkinyl, das geradkettig oder verzweigt ist und, je nach Kettenlänge, ein oder zwei Dreifachbindungen enthält, oder
4) $(C_0-C_{10})$Alkyl-C(O)-O-$(C_1-C_6)$-Alkyl, worin Alkyl gerade oder verzweigt ist und unsubstituiert oder ein- bis dreifach substituiert ist, bevorzugt durch Halogen, Hydroxyl, Amino oder Nitro,
wobei die oben unter 1) bis 4) genannten Reste unsubstituiert sind oder ein-, zwei- oder dreifach substituiert sind unabhängig voneinander durch

a) -OH,
b) Halogen, wie Fluor, Chlor, Brom oder Jod,
c) $-NO_2$ oder
d) $-NH_2$

steht.

19. Verfahren gemäß Anspruch 17 oder 18, **dadurch gekennzeichnet, dass**

a) eine Carbonylverbindung in Anwesenheit der Oxidoreduktase gemäß einem der Ansprüche 1 bis 8, NADPH und Wasser zur entsprechenden chiralen Hydroxyverbindung reduziert,
b) das durch die Oxidoreduktase gebildete NADP mit einem Cosubstrat zu NADPH reduziert, und
c) die gebildete chirale Hydroxyverbindung isoliert wird.

20. Verfahren gemäß Anspruch 17 oder 18, **dadurch gekennzeichnet, dass**

a) eine Carbonylverbindung in Anwesenheit der Oxidoreduktase gemäß einem der Ansprüche 1 bis 8, NADPH und Wasser zur entsprechenden chiralen Hydroxyverbindung reduziert,
b) das durch die Oxidoreduktase gebildete NADP mit einer Dehydrogenase und einem Cosubstrat zu NADPH reduziert, und
c) die gebildete chirale Hydroxyverbindung isoliert wird.

21. Verfahren gemäß Anspruch 17 oder 18, **dadurch gekennzeichnet, dass**

a) eine Carbonylverbindung in Anwesenheit der Oxidoreduktase gemäß einem der Ansprüche 1 bis 8, NADPH und Wasser zur entsprechenden chiralen Hydroxyverbindung reduziert,
b) die Reaktion in Anwesenheit eines organischen Lösungsmittels durchgeführt und
c) die gebildete chirale Hydroxyverbindung isoliert wird.

**22.** Verfahren gemäß Anspruch 17 oder 18, **dadurch gekennzeichnet, dass**

a) eine Carbonylverbindung in Anwesenheit der Oxidoreduktase gemäß einem der Ansprüche 1 bis 8, NADPH und Wasser zur entsprechenden chiralen Hydroxyverbindung reduziert,
b) die Reaktion in Anwesenheit eines organischen Lösungsmittels durchgeführt,
c) das durch die Oxidoreduktase gebildete NADP mit einem Cosubstrat zu NADPH reduziert, und
d) die gebildete chirale Hydroxyverbindung isoliert wird.

**23.** Verfahren gemäß Anspruch 17 oder 18, **dadurch gekennzeichnet, dass**

a) eine Carbonylverbindung in Anwesenheit der Oxidoreduktase gemäß einem der Ansprüche 1 bis 8, NADPH und Wasser zur entsprechenden chiralen Hydroxyverbindung reduziert,
b) das durch die Oxidoreduktase gebildete NADP mit einer Dehydrogenase und einem Cosubstrat gleichzeitig zu NADPH reduziert,
c) die Reaktion in Anwesenheit eines organischen Lösungsmittels durchgeführt und
d) die gebildete chirale Hydroxyverbindung isoliert wird.

**24.** Verfahren gemäß Anspruch 19, 20, 22 oder 23, **dadurch gekennzeichnet, daß** als Cosubstrat 2-Propanol, 2-Butanol, 2-Pentanol oder 2-Octanol verwendet wird.

**25.** Verfahren gemäß einem der Ansprüche 17 bis 24, **dadurch gekennzeichnet, dass** 2-Oxo-4-phenyl-buttersäureethylester zu (R)-2-Hydroxy-4-phenylbuttersäureethylester, 8-Chlor-6-oxo-octansäureethylester zu (R)-8-Chlor-6-hydroxy-octansäureethylester, 2-Chlor-1-phenyl-ethan-1-on zu (1R)-2-Chlor-1-phenyl-ethan-1-ol oder Ethylphenylglyoxylat zu (1R)-Ethylmandelat umsetzt wird.

**26.** Verfahren gemäß Anspruch 20 oder 23, **dadurch gekennzeichnet, dass** eine Dehydrogenase aus Thermoanaerobium brockii, Lactobacillus kefir oder Lactobacillus brevis einsetzt wird.

**27.** Verfahren gemäß Anspruch 20 oder 23, **dadurch gekennzeichnet, dass** als Dehydrogenase NADPH abhängige Formiat-Dehydrogenase und als Cosubstrat ein Salz der Ameisensäure wie Ammoniumformiat, Natriumformiat oder Calciumformiat einsetzt werden.

**28.** Verfahren gemäß einem der Ansprüche 21 bis 23, **dadurch gekennzeichnet, dass** als organisches Lösungsmittel tert.-Butylmethylether, Diisopropylether, Heptan, Hexan oder Cyclohexan oder deren Gemische einsetzt werden.

**29.** Verfahren gemäß einem der Ansprüche 21 bis 23, **dadurch gekennzeichnet, daß** das organische Lösemittel eine Phase bildet, deren Anteil 5 bis 80 %, bevorzugt 10 bis 40 %, bezogen auf das gesamte Reaktionsvolumen, beträgt.

**Claims**

**1.** An oxidoreductase which reduces a carbonyl compound to the corresponding chiral hydroxy compound in the presence of NADPH and water, **characterized in that** more than 70% of its amino acids are identical to the amino acid sequence SEQ ID NO: 13 and that it has a specific activity of more than 1 $\mu$mol per mg of protein, based on the reaction of 2-oxo-4-phenyl-ethyl-butyrate to (R)-2-hydroxy-4-phenyl-ethyl-butyrate.

**2.** The oxidoreductase according to claim 1, **characterized in that** 80% to 99.5%, preferably 90% to 99.5%, particularly preferably 99% to 99.5%, of the amino acids are identical to the amino acid sequence SEQ ID NO: 13.

**3.** The oxidoreductase according to claim 1 or 2, **characterized in that** it comprises the amino acid sequence SEQ ID NO: 13 and is encoded by the DNA sequence SEQ ID NO: 12.

**4.** The oxidoreductase according to any of claims 1 to 3, **characterized in that** it is obtainable from yeasts of the genus Metschnikowia, in particular from Metschnikowia zobellii.

**5.** The oxidoreductase according to any of claims 1 to 4, **characterized in that** it has, in addition, 1 to 40 amino acids more than or 1 to 40 amino acids less than the amino acid sequence SEQ ID NO: 13 and that it has a specific activity of more than 1 μmol per mg of protein, based on the reaction of 2-oxo-4-phenyl-ethyl-butyrate to (R)-2-hydroxy-4-phenylethyl-butyrate.

**6.** The oxidoreductase according to claim 5, **characterized in that** it has 1 to 25 amino acids, in particular 2 to 20 amino acids, particularly preferably 3 to 10 amino acids, more or less than the amino acid sequence SEQ ID NO: 13.

**7.** The oxidoreductase according to any of claims 1 to 6, **characterized in that** it comprises the amino acid sequence SEQ ID NO: 13, is modified once, twice, three, four or five times by a water-soluble polymer and shows a specific activity of more than 1 μmol per mg of protein, based on the reaction of 2-oxo-4-phenyl-ethyl-butyrate to (R)-2-hydroxy-4-phenyl-ethyl-butyrate.

**8.** The oxidoreductase according to claim 7, **characterized in that** the water-soluble polymer is polyethylene glycol.

**9.** An antibody, **characterized in that** it binds specifically to the oxidoreductase according to SEQ ID NO: 13.

**10.** A DNA sequence which codes for the oxidoreductase according to SEQ ID NO: 13.

**11.** A DNA sequence which codes for an oxidoreductase which catalyzes the reduction of a carbonyl compound to the corresponding chiral hydroxy compound in the presence of NADPH and water, wherein the DNA sequence

a) corresponds to SEQ ID NO: 12 or to the complementary strand thereof, or
b) is a DNA sequence which hybridizes to the DNA sequence according to a) or to the complementary strand thereof, with the hybridization taking place under stringent conditions, or
c) is a DNA sequence which, as a result of the degeneration of the genetic code, encodes a protein as it is also encoded by a DNA sequence according to a) or b).

**12.** A DNA sequence, **characterized in that** more than 70% of the nucleic acid bases therein are identical to the DNA sequence SEQ ID NO: 12 or to the complementary strand thereof and that it encodes an oxidoreductase which has a specific activity of more than 1 μmol per mg of protein, based on the reaction of 2-oxo-4-phenyl-ethyl-butyrate to (R)-2-hydroxy-4-phenyl-ethyl-butyrate.

**13.** The DNA sequence according to claim 12, **characterized in that** 80% to 99.5%, preferably 90% to 99.5%, particularly preferably 99% to 99.5%, of the nucleic acid bases are identical to the DNA sequence SEQ ID NO: 12.

**14.** A cloning vector, **characterized in that** it comprises one or several of the DNA sequences according to any of claims 10 to 13.

**15.** An expression vector, **characterized in that** it comprises one or several of the DNA sequences according to any of claims 10 to 13 and is linked to an expression control sequence.

**16.** A host cell which is a bacterial, yeast, insect or plant cell and has been transformed or transfected with an expression vector according to claim 15.

**17.** A process for the enantioselective reduction of carbonyl compounds to the corresponding chiral hydroxy compounds, **characterized in that**

a) a carbonyl compound is reduced to the corresponding chiral hydroxy compound in the presence of an oxidoreductase according to any of claims 1 to 8, NADPH and water, and
b) the chiral hydroxy compound formed is isolated.

**18.** A process according to claim 17, **characterized in that** a compound of Formula I

$$R1\text{-}C(O)\text{-}R2 \qquad (I)$$

is used as the carbonyl compound,
wherein R1 stands for

1) $(C_1-C_{20})$-alkyl, which is linear-chain or branched,

2) $(C_2-C_{20})$-alkenyl, which is linear-chain or branched and contains one, two, three or four double bonds, depending on the chain length,

3) $(C_2-C_{20})$-alkynyl, which is linear-chain or branched and contains one, two, three or four triple bonds, depending on the chain length,

4) $(C_6-C_{14})$-aryl,

5) $(C_1-C_8)$-alkyl-$(C_6-C_{14})$-aryl,

6) $(C_5-C_{14})$-heterocycle which is unsubstituted or substituted one to three times, preferably by halogen, hydroxyl, amino or nitro, or

7) $(C_3-C_7)$-cycloalkyl,

wherein the moieties mentioned above under 1) to 7) are unsubstituted or substituted one, two or three times, independently of each other, by

  a) -OH,
  b) halogen such as fluorine, chlorine, bromine or iodine,
  c) $-NO_2$ or
  d) $-NH_2$, and

R2 stands for

1) $(C_1-C_6)$-alkyl, which is linear-chain or branched,

2) $(C_2-C_6)$-alkenyl, which is linear-chain or branched and contains one, two or three double bonds, depending on the chain length,

3) $(C_2-C_6)$-alkynyl, which is linear-chain or branched and contains one or two triple bonds, depending on the chain length,

4) $(C_0-C_{10})$-alkyl-C(O)-O-$(C_1-C_6)$-alkyl, wherein alkyl is linear or branched and is unsubstituted or substituted one to three times, preferably by halogen, hydroxyl, amino or nitro,

wherein the moieties mentioned above under 1) to 4) are unsubstituted or substituted one, two or three times, independently of each other, by

  a) -OH,
  b) halogen such as fluorine, chlorine, bromine or iodine,
  c) $-NO_2$ or
  d) $-NH_2$.

**19.** The process according to claim 17 or 18, **characterized in that**

  a) a carbonyl compound is reduced to the corresponding chiral hydroxy compound in the presence of the oxidoreductase according to any of claims 1 to 8, NADPH and water,
  b) the NADP formed by the oxidoreductase is reduced to NADPH with a cosubstrate, and
  c) the chiral hydroxy compound formed is isolated.

**20.** The process according to claim 17 or 18, **characterized in that**

  a) a carbonyl compound is reduced to the corresponding chiral hydroxy compound in the presence of the oxidoreductase according to any of claims 1 to 8, NADPH and water,
  b) the NADP formed by the oxidoreductase is reduced to NADPH with a dehydrogenase and a cosubstrate, and
  c) the chiral hydroxy compound formed is isolated.

**21.** The process according to claim 17 or 18, **characterized in that**

  a) a carbonyl compound is reduced to the corresponding chiral hydroxy compound in the presence of the oxidoreductase according to any of claims 1 to 8, NADPH and water,
  b) the reaction is carried out in the presence of an organic solvent, and
  c) the chiral hydroxy compound formed is isolated.

**22.** The process according to claim 17 or 18, **characterized in that**

a) a carbonyl compound is reduced to the corresponding chiral hydroxy compound in the presence of the oxidoreductase according to any of claims 1 to 8, NADPH and water,
b) the reaction is carried out in the presence of an organic solvent,
c) the NADP formed by the oxidoreductase is reduced to NADPH with a cosubstrate, and
d) the chiral hydroxy compound formed is isolated.

23. The process according to claim 17 or 18, **characterized in that**

a) a carbonyl compound is reduced to the corresponding chiral hydroxy compound in the presence of the oxidoreductase according to any of claims 1 to 8, NADPH and water,
b) the NADP formed by the oxidoreductase is simultaneously reduced to NADPH with a dehydrogenase and a cosubstrate,
c) the reaction is carried out in the presence of an organic solvent, and
d) the chiral hydroxy compound formed is isolated.

24. The process according to claim 19, 20, 22 or 23, **characterized in that** 2-propanol, 2-butanol, 2-pentanol or 2-octanol is used as the cosubstrate.

25. The process according to any of claims 17 to 24, **characterized in that** 2-oxo-4-phenyl-ethyl-butyrate is reacted to (R)-2-hydroxy-4-phenyl-ethyl-butyrate, 8-chloro-6-oxoethyl-caprylate is reacted to (R)-8-chloro-6-hydroxy-ethyl-caprylate, 2-chloro-1-phenylethane-1-one is reacted to (1R)-2-chloro-1-phenyl-ethane-1-ol or ethyl phenyl glyoxylate is reacted to (1R)-ethyl mandelate.

26. The process according to claim 20 or 23, **characterized in that** a dehydrogenase from Thermoanaerobium brockii, Lactobacillus kefir or Lactobacillus brevis is used.

27. The process according to claim 20 or 23, **characterized in that** NADPH-dependent formate dehydrogenase is used as the dehydrogenase and a salt of formic acid such as ammonium formate, sodium formate or calcium formate is used as the cosubstrate.

28. The process according to any of claims 21 to 23, **characterized in that** tertiary butyl methyl ether, diisopropyl ether, heptane, hexane or cyclohexane or mixtures thereof is/are used as the organic solvent.

29. The process according to any of claims 21 to 23, **characterized in that** the organic solvent forms a phase the proportion of which is 5% to 80%, preferably 10 to 40%, based on the total reaction volume.

## Revendications

1. Oxydoréductase qui réduit, en présence de NADPH et d'eau, un composé carbonyle en composé hydroxy chiral correspondant, **caractérisée en ce que** plus de 70 % de ses acides aminés sont identiques à la séquence d'acides aminés SEQ ID N° : 13 et **en ce qu'**elle présente une activité spécifique de plus de 1 $\mu$mole par mg de protéine, par rapport à la conversion de l'acide 2-oxo-4-phényl-butyrique éthylester en acide (R)-2-hydroxy-4-phényl-butyrique éthylester.

2. Oxydoréductase selon la revendication 1, **caractérisée en ce que** 80 à 99,5 %, de préférence 90 à 99,5 %, de manière particulièrement préférée, 99 à 99,5 % des acides aminés sont identiques à la séquence d'acides aminés SEQ ID N° : 13.

3. Oxydoréductase selon la revendication 1 ou 2, **caractérisée en ce qu'**elle présente la séquence d'acides aminés SEQ ID N° : 13 et est codée par la séquence d'ADN SEQ ID N° : 12.

4. Oxydoréductase selon l'une des revendications 1 à 3, **caractérisée en ce qu'**elle peut être obtenue à partir de levures du genre Metschnikowia, en particulier, Metschnikowia zobellii.

5. Oxydoréductase selon l'une des revendications 1 à 4, **caractérisée en ce qu'**elle présente 1 à 40 acides aminés en plus ou 1 à 40 acides aminés en moins par rapport à la séquence d'acides aminés SEQ ID N° : 13, et **en ce qu'**elle présente une activité spécifique de plus de 1 $\mu$mole par mg de protéine, par rapport à la conversion de l'acide 2-oxo-4-phénylbutyrique éthylester en acide (R)-2-hydroxy-4-phényl-butyrique éthylester.

**6.** Oxydoréductase selon la revendication 5, **caractérisée en ce qu'**elle présente 1 à 25 acides aminés, en particulier, 2 à 20 acides aminés, de manière particulièrement préférée, 3 à 10 acides aminés, de plus ou de moins que la séquence d'acides aminés SEQ ID N° : 13.

**7.** Oxydoréductase selon l'une des revendications 1 à 6, **caractérisée en ce qu'**elle présente la séquence d'acides aminés SEQ ID N° : 13, est modifiée une, deux, trois, quatre ou cinq fois par un polymère hydrosoluble et présente une activité spécifique de plus de 1 $\mu$mole par mg de protéine, par rapport à la conversion de l'acide 2-oxo-4-phénylbutyrique éthylester en acide (R)-2-hydroxy-4-phényl-butyrique éthylester.

**8.** Oxydoréductase selon la revendication 7, **caractérisée en ce que** le polymère hydrosoluble est le polyéthylène glycol.

**9.** Anticorps **caractérisé en ce qu'**il se lie de façon spécifique à l'oxydoréductase selon SEQ ID N° : 13.

**10.** Séquence d'ADN qui code pour l'oxydoréductase selon SEQ ID N° : 13.

**11.** Séquence d'ADN qui code pour une oxydoréductase qui catalyse, en présence de NADPH et d'eau, la réduction d'un composé carbonyle en composé hydroxy chiral correspondant, la séquence d'ADN

a) correspondant à SEQ ID N° : 12 ou son brin complémentaire ou
b) étant une séquence d'ADN qui s'hybride avec la séquence d'ADN selon le point a) ou son brin complémentaire, l'hybridation s'effectuant dans des conditions stringentes, ou
c) étant une séquence d'ADN qui, en raison de la dégénérescence du code génétique, code pour une protéine telle que codée également par une séquence d'ADN selon le point a) ou b).

**12.** Séquence d'ADN, **caractérisée en ce que** dans celle-ci, plus de 70 % des bases d'acide nucléique sont identiques à la séquence d'ADN SEQ ID N° : 12 ou son brin complémentaire, et **en ce qu'**elle code pour une oxydoréductase qui présente une activité spécifique de plus de 1 $\mu$mole par mg de protéine, par rapport à la conversion de l'acide 2-oxo-4-phénylbutyrique éthylester en acide (R)-2-hydroxy-4-phényl-butyrique éthylester.

**13.** Séquence d'ADN selon la revendication 12, **caractérisée en ce que** 80 % à 99,5 %, de préférence, 90 % à 99,5 %, de manière particulièrement préférée, 99 % à 99,5 % des bases d'acides nucléiques sont identiques à la séquence d'ADN SEQ ID N° : 12.

**14.** Vecteur de clonage, **caractérisé en ce qu'**il comprend une ou plusieurs séquences d'ADN selon l'une des revendications 10 à 13.

**15.** Vecteur d'expression, **caractérisé en ce qu'**il comprend une ou plusieurs séquences d'ADN selon l'une des revendications 10 à 13, et est lié à une séquence de contrôle de l'expression.

**16.** Cellule hôte qui est une cellules bactérienne, une cellule de levure, d'insecte ou de plante et a été transformée ou transfectée avec un vecteur d'expression selon la revendication 15.

**17.** Procédé de réduction énantiosélective de composés carbonyle en composés hydroxy chiraux correspondants, **caractérisé en ce que**

a) un composé carbonyle est réduit en présence d'une oxydoréductase selon l'une des revendications 1 à 8, de NADPH et d'eau en composé hydroxy chiral correspondant, et
b) le composé hydroxy chiral formé est isolé.

**18.** Procédé selon la revendication 17, **caractérisé en ce que** l'on utilise comme composé carbonyle, un composé de formule I

R1-C(O)-R2      (I)

dans laquelle R1 désigne

1) un groupe alkyle (en $C_1$ à $C_{20}$) qui est linéaire ou ramifié,

2) un groupe alcényle (en $C_2$ à $C_{20}$) qui est linéaire ou ramifié et contient, en fonction de la longueur de chaîne, une, deux, trois ou quatre liaisons doubles,

3) un groupe alcinyle (en $C_2$ à $C_{20}$) qui est linéaire ou ramifié et en fonction de la longueur de chaîne, contient un, deux, trois ou quatre liaisons triples,

4) un groupe aryle (en $C_6$ à $C_{14}$),

5) un groupe alkyle (en $C_1$ à $C_8$)-aryle (en $C_6$ à $C_{14}$),

6) un hétérocycle (en $C_5$ à $C_{14}$) qui est non substitué ou mono- à trisubstitué, de préférence par un atome d'halogène, un groupe hydroxyle, amino ou nitro, ou

7) un groupe cycloalkyle (en $C_3$ à $C_7$),

dans laquelle les radicaux mentionnés ci-dessus des points 1) à 7) sont non substitués ou mono-, di- ou trisubstitués indépendamment les uns des autres par

a) -OH,

b) un atome d'halogène tel que le fluor, le chlore, le brome ou l'iode,

c) $-NO_2$ ou

d) $-NH_2$ et

R2 désigne

1) un groupe alkyle (en $C_1$ à $C_6$) qui est linéaire ou ramifié,

2) un groupe alcényle (en $C_2$ à $C_6$) qui est linéaire ou ramifié et contient, en fonction de la longueur de chaîne, une, deux ou trois liaisons doubles,

3) un groupe alcinyle (en $C_2$ à $C_6$) qui est linéaire ou ramifié et en fonction de la longueur de chaîne, contient une ou deux liaisons triples, ou

4) un groupe alkyle (en $C_0$ à $C_{10}$)-C(O)-O-alkyle en ($C_1$ à $C_6$), le groupe alkyle étant linéaire ou ramifié et non substitué ou mono- à trisubstitué, de préférence par un atome d'halogène, un groupe hydroxyle, amino ou nitro,

dans laquelle les radicaux mentionnés ci-dessus des points 1) à 4) sont non substitués ou mono-, di- ou trisubstitués indépendamment les uns des autres par

a) -OH,

b) un atome d'halogène tel que le fluor, le chlore, le brome ou l'iode,

c) $-NO_2$ ou

d) $-NH_2$.

**19.** Procédé selon la revendication 17 ou 18, **caractérisé en ce que**

a) un composé carbonyle est réduit en présence de l'oxydoréductase selon l'une des revendications 1 à 8, de NADPH et d'eau en composé hydroxy chiral correspondant,

b) le NADP formé par l'oxydoréductase est réduit avec un co-substrat en NADPH, et

c) le composé hydroxy chiral formé est isolé.

**20.** Procédé selon la revendication 17 ou 18, **caractérisé en ce que**

a) un composé carbonyle est réduit en présence de l'oxydoréductase selon l'une des revendications 1 à 8, de NADPH et d'eau en composé hydroxy chiral correspondant,

b) le NADP formé par l'oxydoréductase est réduit avec une déshydrogénase et un co-substrat en NADPH, et

c) le composé hydroxy chiral formé est isolé.

**21.** Procédé selon la revendication 17 ou 18, **caractérisé en ce que**

a) un composé carbonyle est réduit en présence de l'oxydoréductase selon l'une des revendications 1 à 8, de NADPH et d'eau en composé hydroxy chiral correspondant,

b) la réaction est réalisée en présence d'un solvant organique et

c) la composé hydroxy chiral formé est isolé.

**22.** Procédé selon la revendication 17 ou 18, **caractérisé en ce que**

a) un composé carbonyle est réduit en présence de l'oxydoréductase selon l'une des revendications 1 à 8, de NADPH et d'eau en composé hydroxy chiral correspondant,
b) la réaction est réalisée en présence d'un solvant organique,
c) le NADP formé par l'oxydoréductase est réduit avec un co-substrat en NADPH, et
d) le composé hydroxy chiral formé est isolé.

**23.** Procédé selon la revendication 17 ou 18, **caractérisé en ce que**

a) un composé carbonyle est réduit en présence de l'oxydoréductase selon l'une des revendications 1 à 8, de NADPH et d'eau en composé hydroxy chiral correspondant,
b) le NADP formé par l'oxydoréductase est réduit en même temps avec une déshydrogénase et un co-substrat en NADPH,
c) la réaction est réalisée en présence d'un solvant organique et
d) le composé hydroxy chiral formé est isolé.

**24.** Procédé selon la revendication 19, 20, 22 ou 23,
**caractérisé en ce que** l'on utilise comme co-substrat, le 2-propanol, le 2-butanol, le 2-pentanol ou le 2-octanol.

**25.** Procédé selon l'une des revendications 17 à 24, **caractérisé en ce que** l'acide 2-oxo-4-phényl-butyrique éthyl ester est converti en acide (R)-2-hydroxy-4-phénylbutyrique éthylester, l'acide 8-chloro-6-oxo-octanoïque éthylester est converti en acide (R)-8-chloro-6-hydroxy-octanoïque éthylester, le 2-chloro-1-phényléthan-1-one est converti en (1R)-2-chloro-1-phényl-éthan-1-ol ou l'éthylphénylglyoxylate est converti en (1R)-éthylmandélate.

**26.** Procédé selon la revendication 20 ou 23, **caractérisé en ce que** l'on utilise une déshydrogénase de Thermoanaerobium brockii, de Lactobacillus kefir ou de Lactobacillus brevis.

**27.** Procédé selon la revendication 20 ou 23, **caractérisé en ce que** l'on utilise comme déshydrogénase, la déshydrogénase de formiate dépendante du NADPH et comme co-substrat, un sel d'acide formique tel que le formiate d'ammonium, le formiate de sodium ou le formiate de calcium.

**28.** Procédé selon l'une des revendications 21 à 23, **caractérisé en ce que** l'on utilise comme solvant organique, le tert.-butylméthyléther, le diisopropyléther, l'heptane, l'hexane ou le cyclohexane ou leurs mélanges.

**29.** Procédé selon l'une des revendications 21 à 23, **caractérisé en ce que** le solvant organique forme une phase dont la proportion est de 5 à 80 %, de préférence de 10 à 40 % par rapport au volume réactionnel total.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5523223 A
- US 5763236 A
- DE 10327454
- US 5200335 A
- DE 19610984 A1
- DE 10119274
- US 05385833 A

- WO 9835025 A
- JP HEI11187869 A
- EP 0918090 A
- DE 19610984
- EP 0456107 A
- WO 9732012 A

**In der Beschreibung aufgeführte Nicht-Patentliteratur**

- *Enzyme Engineering,* 1982, vol. 6, 107
- Enzymes. Academic Press, 1963, 25-83
- *Biosci. Biotechnol. Biochem.,* 1999, vol. 63 (10), 1721-1729
- *Biochim,. Biophys. Acta,* 1982, vol. 716, 298-307
- *FEMS Microbiology Letters,* 1999, vol. 170, 31-39
- *FEMS Microbiology Letters,* 1990, vol. 70, 45-48

- **Von Costello C.A. et al.** *Eur. J. Biochem.,* 2000, vol. 267, 5493-5501
- **Sambrok ; Russel.** Molecular Cloning a laboratory Manual. vol. 1, 30-32
- **Tishkov et al.** *J. Biotechnol. Bioeng.,* 1999, vol. 64, 187-193
- **Lowry et al.** *Journal of Biological Chemistry,* 1951, vol. 193, 265-275
- **Peterson et al.** *Analytical Biochemistry,* 1979, vol. 100, 201-220

SEQUENCE LISTING

<110> Juelich Enzyme Products GmbH

<120> Oxidoreduktase aus Metschnikowia zobellii

<130> Metschnikowia

<160> 17

<170> PatentIn version 3.1

<210> 1
<211> 11
<212> PRT
<213> Metschnikowia zobellii

<400> 1

```
Glu Leu Leu Leu Pro Ala Val Glu Gly Thr Lys
1               5                   10
```

<210> 2
<211> 21
<212> PRT
<213> Metschnikowia zobellii

<400> 2

Leu Gly Pro Asn Asp Glu Val Pro Ser Gln Ser His Trp Gly Ser Cys
1               5               10                  15

Gly Phe Val Asp Val
            20

<210> 3
<211> 21
<212> PRT
<213> Artificial

<400> 3

Cys Cys Asn Gly Cys His Gly Thr Asp Gly Ala Ala Gly Gly Trp Ala .
1               5               10                  15

Cys Trp Ala Ala Arg
            20

<210> 4
<211> 21
<212> PRT
<213> Artificial

<400> 4

Tyr Thr Thr Trp Gly Thr Trp Cys Cys Thr Thr Cys His Ala Cys Asp
1               5               10                  15

Gly Cys Met Gly Gly
            20

<210> 5
<211> 20
<212> PRT
<213> Artificial

<400> 5

Gly Gly Asn Cys Cys Asn Ala Ala Tyr Gly Ala Thr Gly Ala Arg Gly
1               5               10                  15

Thr Asn Cys Cys
            20

<210> 6
<211> 20
<212> PRT
<213> Artificial

<400> 6


Gly Gly Asn Ala Cys Asn Thr Cys Ala Thr Cys Arg Thr Thr Asn Gly
1                   5                   10                  15


Gly Arg Cys Cys
                20


<210> 7
<211> 451
<212> DNA
<213> Metschnikowia zobellii

<400> 7


ttaattaacc   ctcactaaag   ggaacaaaag   ctggagctcc   accgcggtgg
cggccgctct      60

agaactagtg   gatcccccgg   gctgcaggaa   ttcggcacga   ggctcaaact
tcatctgctc     120

tacctccaca   acttatcatg   accacttcag   tctttgtctc   tggtgccacc
ggcttcattg     180


cccagcacgt   cgtcaaactc   cttctctcga   aaggctacaa   cgtcgttggc
tccgtcagaa     240

ccgccgagaa   aggcaagaac   ctagcaaagc   tatttggcac   cggctccttc
acctacgagg     300

tggtgcccaa   actcgaagcg   cctggtgcct   ttgacgaggc   cttggaaaag
catccagagg     360

tgtctgtgtt   tttgcacacg   gcctcgcccg   tcacctttga   cgtcaaggac
attgagaaag     420

aattgcttct   ccccgccgta   gaaggaacaa   a
             451


<210> 8
<211> 484
<212> DNA
<213> Metschnikowia zobellii

<400> 8

```
aattcggctt   ggtccgaatg   atgaagttcc   ctcgcaactg   gggggtttcg
tcgacgtgag        60

agacgtggcc   aaggctcact   tggccgcatt   cgagggcgga   ctctcgaacc
agcgcctttt       120

gcttagaacg   gcagcgttta   atgcgcaacg   cgtgcttgac   ataatcaaca
ataagtttgt       180

taacttgaga   ggacagttgc   ccactggaac   gccttgtaaa   ggcgagcctg
aaagcactgg       240

ctctgtgacg   gacaattcga   gaaccaagaa   gttattgaac   ttcccagcca
tcgacttgga       300

gaactgtgtg   gtggactcgg   ttacccagct   catgaagtcc   cagaagaagg
ttttgtaagc       360

gcacatagtg   aatcaaaaag   tacagtgaca   aaaaaaaaa    aaaaaaaac
tcgagggggg       420

gcccggtacc   caattcgccc   tatagtgagt   cgtattaaag   ccgaattcca
gcacactggc       480

ggcc
        484
```
.

```
<210> 9
<211> 36
<212> DNA
<213> Artificial

<400> 9
```

```
ggaattccat   atgatgacca   cttcagtctt   tgtctc
       36
```

```
<210> 10
<211> 30
<212> DNA
<213> Artificial

<400> 10
```

```
cccaagcttc   gcttacaaaa   ccttcttctg
       30
```

```
<210> 11
<211> 33
<212> DNA
<213> Artificial

<400> 11
```

```
               gcgcggatcc atgaccactt cagtctttgt ctc
                    33
```

<210> 12
<211> 1155
<212> DNA
<213> Metschnikowia zobellii

<400> 12

```
tgagcggata  caattcccct  ctagaaataa  ttttgtttaa  ctttaagaag
gagatataca      60

tatgatgacc  acttcagtct  ttgtctctgg  tgccaccggc  ttcattgccc
agcacgtcgt     120

caaactcctt  ctctcgaaag  gctacaacgt  cgttggctcc  gtcagaaccg
ccgagaaagg     180

caagaaccta  gcaaagctat  ttggcaccgg  ctccttcacc  tacgaggtgg
tgcccaaact     240

cgaagcgcct  ggtgcctttg  acgaggcctt  ggaaaagcat  ccagaggtgt
ctgtgttttt     300

gcacacggcc  tcgcccgtca  cctttgacgt  caaggacatt  gagaaagagt
tgcttctccc     360

cgccgtcgag  ggcaccaaaa  acgtctttag  tgccatcaaa  gcccacggcc
cgcagatcaa     420

aaacgtcgtg  gtgacgtcct  ctgttgctgc  ggcactcgat  cccgctagaa
acctggaccc     480

cacgttcact  gtgaatgaag  actcttggaa  cccaatctcg  tgggaggact
cgaagcagaa     540

cgccatgacc  gggtactttg  gctccaagaa  gtttgcagag  aaggccgcgt
gggattttgt     600

ggaagccgag  aagcccaact  ttctgttgaa  caccgtgttg  cccgtgtacg
```

```
tctttggccc        660

ccaggcgttt   gactccgagg   tcaagggcga   gctcaactac   tctgccgaaa
tcatcaacaa        720

gttgttgaag   ttgggaccca   acgacgaagt   gccctcgcaa   ctggggggtt
tcgtcgacgt        780

gagagacgtg   gccaaggctc   acttggccgc   attcgagggc   ggactctcga
accagcgcct        840

tttgcttaga   acggcagcgt   ttaatgcgca   acgcgtgctt   gacataatca
acaataagtt        900

tgttaacttg   agaggacagt   tgcccactgg   aacgccttgt   aaaggcgagc
ctgaaagcac        960

tggctctgtg   acggacaatt   cgagaaccaa   gaagttattg   aacttcccag
ccatcgactt        1020

ggagaactgt   gtggtggact   cggttaccca   gctcatgaag   tcccagaaga
aggttttgta        1080

agcgaagctt   gcggccgcac   tcgagcacca   ccaccaccac   cactgagatc
cggctgctaa        1140

caaagcccga   aagga
          1155


<210> 13
<211> 338
<212> PRT
<213> Metschnikowia zobellii

<400> 13


Met Thr Thr Ser Val Phe Val Ser Gly Ala Thr Gly Phe Ile Ala Gln
1               5               10              15


His Val Val Lys Leu Leu Leu Ser Lys Gly Tyr Asn Val Val Gly Ser
                20              25              30


Val Arg Thr Ala Glu Lys Gly Lys Asn Leu Ala Lys Leu Phe Gly Thr
            35              40              45


Gly Ser Phe Thr Tyr Glu Val Val Pro Lys Leu Glu Ala Pro Gly Ala
        50              55              60


Phe Asp Glu Ala Leu Glu Lys His Pro Glu Val Ser Val Phe Leu His
65              70              75              80
```

Thr Ala Ser Pro Val Thr Phe Asp Val Lys Asp Ile Glu Lys Glu Leu
                85                      90                      95

Leu Leu Pro Ala Val Glu Gly Thr Lys Asn Val Phe Ser Ala Ile Lys
            100                 105                 110

Ala His Gly Pro Gln Ile Lys Asn Val Val Val Thr Ser Ser Val Ala
        115                 120                 125

Ala Ala Leu Asp Pro Ala Arg Asn Leu Asp Pro Thr Phe Thr Val Asn
    130                 135                 140

Glu Asp Ser Trp Asn Pro Ile Ser Trp Glu Asp Ser Lys Gln Asn Ala
145                 150                 155                 160

Met Thr Gly Tyr Phe Gly Ser Lys Lys Phe Ala Glu Lys Ala Ala Trp
            165                 170                 175

Asp Phe Val Glu Ala Glu Lys Pro Asn Phe Leu Leu Asn Thr Val Leu
            180                 185                 190

Pro Val Tyr Val Phe Gly Pro Gln Ala Phe Asp Ser Glu Val Lys Gly
        195                 200                 205

Glu Leu Asn Tyr Ser Ala Glu Ile Ile Asn Lys Leu Leu Lys Leu Gly
    210                 215                 220

Pro Asn Asp Glu Val Pro Ser Gln Leu Gly Gly Phe Val Asp Val Arg
225                 230                 235                 240

Asp Val Ala Lys Ala His Leu Ala Ala Phe Glu Gly Gly Leu Ser Asn
            245                 250                 255

Gln Arg Leu Leu Leu Arg Thr Ala Ala Phe Asn Ala Gln Arg Val Leu
            260                 265                 270

Asp Ile Ile Asn Asn Lys Phe Val Asn Leu Arg Gly Gln Leu Pro Thr
        275                280                285

Gly Thr Pro Cys Lys Gly Glu Pro Glu Ser Thr Gly Ser Val Thr Asp
        290                295                300

Asn Ser Arg Thr Lys Lys Leu Leu Asn Phe Pro Ala Ile Asp Leu Glu
305                310                315                320

Asn Cys Val Val Asp Ser Val Thr Gln Leu Met Lys Ser Gln Lys Lys
                325                330                335

Val Leu


<210> 14
<211> 20
<212> DNA
<213> Artificial

<400> 14

                        aattaaccct cactaaaggg
                                20


<210> 15
<211> 20
<212> DNA
<213> Artificial

<400> 15

                        taatacgact cactataggg
                                20


<210> 16
<211> 104
<212> PRT
<213> Metschnikowia zobellii

<400> 16

```
Met Thr Thr Ser Val Phe Val Ser Gly Ala Thr Gly Phe Ile Ala Gln
1               5                   10                  15

His Val Val Lys Leu Leu Leu Ser Lys Gly Tyr Asn Val Val Gly Ser
                20                  25                  30

Val Arg Thr Ala Glu Lys Gly Lys Asn Leu Ala Lys Leu Phe Gly Thr
        35                  40                  45

Gly Ser Phe Thr Tyr Glu Val Val Pro Lys Leu Glu Ala Pro Gly Ala
    50                  55                  60

Phe Asp Glu Ala Leu Glu Lys His Pro Glu Val Ser Val Phe Leu His
65                  70                  75                  80

Thr Ala Ser Pro Val Thr Phe Asp Val Lys Asp Ile Glu Lys Glu Leu
                85                  90                  95

Leu Leu Pro Ala Val Glu Gly Thr
                100
```

<210> 17
<211> 127
<212> PRT
<213> Metschnikowia zobellii

<400> 17

```
Ile Arg Leu Gly Pro Asn Asp Glu Val Pro Ser Gln Leu Gly Gly Phe
1               5                   10                  15

Val Asp Val Arg Asp Val Ala Lys Ala His Leu Ala Ala Phe Glu Gly
                20                  25                  30

Gly Leu Ser Asn Gln Arg Leu Leu Leu Arg Thr Ala Ala Phe Asn Ala
            35                  40                  45

Gln Arg Val Leu Asp Ile Ile Asn Asn Lys Phe Val Asn Leu Arg Gly
        50                  55                  60

Gln Leu Pro Thr Gly Thr Pro Cys Lys Gly Glu Pro Glu Ser Thr Gly
65                  70                  75                  80

Ser Val Thr Asp Asn Ser Arg Thr Lys Lys Leu Leu Asn Phe Pro Ala
                85                  90                  95

Ile Asp Leu Glu Asn Cys Val Val Asp Ser Val Thr Gln Leu Met Lys
                100                 105                 110

Ser Gln Lys Lys Val Leu Ala His Ile Val Asn Gln Lys Val Gln
            115                 120                 125
```